(19) Europäisches Patentamt
European Patent Office
Office européen des brevets

(11) Veröffentlichungsnummer: **0 469 275 A2**

(12) # EUROPÄISCHE PATENTANMELDUNG

(21) Anmeldenummer: **91110026.1**

(51) Int. Cl.[5]: **C07J 71/00**, C07J 7/00

(22) Anmeldetag: **19.06.91**

(30) Priorität: **19.06.90 HU 389690**

(43) Veröffentlichungstag der Anmeldung:
**05.02.92 Patentblatt 92/06**

(84) Benannte Vertragsstaaten:
**AT BE CH DE DK ES FR GB GR IT LI LU NL SE**

(71) Anmelder: **RICHTER GEDEON VEGYESZETI GYAR R.T.**
**Gyömröi ut 19-21**
**H-1475 Budapest X(HU)**

(72) Erfinder: **Toro Andras**
**Repasi J.u 2**
**H-1149 Budapest(HU)**
Erfinder: **Dr. Gabor Ambrus**
**45 b Csalan u**
**H-1025 Budapest(HU)**
Erfinder: **Pallagi Istvan**
**59 Hevesi Gy.u**
**H-1157 Budapest(HU)**
Erfinder: **Dr. Nandor Makk**
**1-2 Helgyalja u.**
**2623 Kismaros(HU)**
Erfinder: **Dr. Gyula Horvath**
**4-6 Kigyo u.**
**H-1052 Budapest(HU)**
Erfinder: **Dr. Ferenc Szederkenyi**
**4 b Veso u.**
**H-1133 Budapest(HU)**
Erfinder: **Dr. Eva Ilkoy**
**37 Munkacsy M. u**
**H-1045 Budapest(HU)**
Erfinder: **Dr. Antonia Jekkel geb. Bokany**
**38 Damjanich u.**
**H-1071 Budapest(HU)**
Erfinder: **Moravcsik Imre**
**38 Mester u.**
**H-1095 Budapest(HU)**
Erfinder: **Dr. Kalman Könczöl**
**16 Dunyov I. u**
**H-1184 Budapest(HU)**

(74) Vertreter: **Beszédes, Stephan G., Dr.**
**Patentanwalt**
**Münchener Strasse 80a Postfach 1168**
**W-8060 Dachau(DE)**

(54) **3-(oxo)- oder 3-(hydroxy)-substituierte 17 Alpha, 20-(Epoxy)-pregnan-20-carbonsäure-beziehungsweise-isocyanat-Derivate, ihre Verwendung und Verfahren zur Herstellung von 3-(oxo)- oder 3-(hydroxy)-substituierten 17 Alpha-(Hydroxy)-20-(oxo)-pregnan-Derivaten und der ersteren.**

(57) Gegenstand der Erfindung sind als Zwischenprodukte einsetzbare 3-(oxo)- oder 3-(hydroxy)-substituierte 17α,20-(Epoxy)-pregnan-20-carbonsäure- beziehungsweise -isocyanat-Derivate der allgemeinen Formel

EP 0 469 275 A2

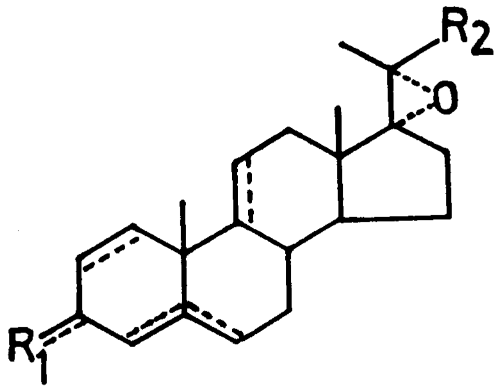

I)

worin

R1 für eine Hydroxy- oder Oxogruppe steht,

R2 eine Carboxylgruppe der Formel

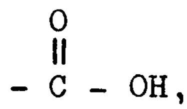

eine Carbonsäureanhydridgruppe, insbesondere der Formel

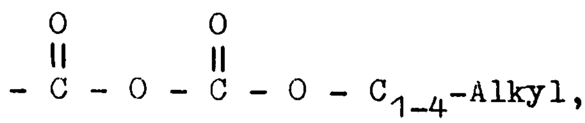

eine Carbonsäureazidogruppe der Formel

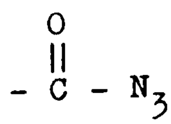

oder eine Isocyanatgruppe der Formel - N = C = O bedeutet und

die gestrichelten Linien gegebenenfalls vorhandene weitere chemische Bindungen darstellen, wobei nicht gleichzeitig in den 4(5)- und 5(6)- Stellungen Doppelbindungen vorliegen können.

Gegenstand der Erfindung ist auch die Verwendung von solchen Verbindungen.

Gegenstand der Erfindung ist auch ein Verfahren zur Herstellung dieser Verbindungen, bei welchem in situ hergestellte 3-(oxo)- oder 3-(hydroxy)-substituierte 17α,20-(Epoxy)-pregnan-20-isocyanat-Derivate oder 3-(oxo)- oder 3-(hydroxy)-substituierte 17α,20-(Epoxy)-pregnan-20-carbonsäureazid-Derivate mit Mineralsauren oder organischen Säuren umgesetzt werden, wobei die genannten Zwischenprodukte durch Umsetzen von 3-(oxo)- oder 3-(hydroxy)-substituierten 23,24-Di-(nor)-17(20)-cholensäure-Derivate in Gegenwart von Sauerstoffsäuren oder Persäuren oder Polysäuren von Übergangsmetallen oder Komplexen derselben und gegebenenfalls Umsetzen der erhaltenen Produkte zu hinsichtlich der Carboxylfunktion reaktionsfähigen Derivaten und gegebenenfalls Umsetzen der letzteren mit Azidionen enthaltenden Salzen und gegebenenfalls Umlagern der erhaltenen 3-(oxo)- oder 3-(hydroxy)-substituierte 17α,20-(Epoxy)-pregnan-20-carbonsäureazid-Derivate in der Wärme zu 17α,20-(Epoxy)-pregnan-20-isocyanat-Derivaten erhältlich sind.

Weiterhin ist Gegenstand der Erfindung ein Verfahren zur Herstellung der genannten Zwischenprodukte.

Die Erfindung betrifft neue 3-(oxo)- oder 3-(hydroxy)-substituierte 17α,20-(Epoxy)-pregnan-20-carbonsäure- beziehungsweise -isocyanat-Derivate, ihre Verwendung und ein Verfahren zur Herstellung von 3-(oxo)- oder 3-(hydroxy)-substituierten 17α-(Hydroxy)-20-(oxo)-pregnan-Derivaten und der ersteren.

Bei der technischen beziehungsweise industriellen Herstellung von Steroiden werden die 17α-(Hydroxy)-20-(oxo)-pregnan-Derivate als Zwischenprodukte für die Herstellung von Hydrocortison-glucocorticoid und von aus diesem entwickelten entzündungshemmenden Corticosteroid-Arzneimitteln, wie Prednisolon, Triamcinolon, Dexamethason und Betamethason, sowie bei der Synthese von gestagen wirkenden 17α-(Hydroxy)-progesteron-17-ester-Derivaten, wie 17α-(Hydroxy)-progesteron-capronat und Chlormadinon-acetat, und des antiandrogen wirkenden Arzneimittelwirkstoffes Cyproteron-acetat verwendet.

Die eine 17α-(Hydroxy)-20-(oxo)-pregnan-Struktur aufweisenden Zwischenprodukte werden technisch beziehungsweise industriell zum Teil durch Umsetzen des durch chemischen Abbau von Diosgenin und Solasodin gewinnbaren 3β-(Acetoxy)-5,16-pregnadien-20-ones (N. Applezweig: Steroid Drugs Vol. 1, S. 56; McGraw Hill Corporation, New York, 1962) und zum Teil aus eine Androstanstruktur aufweisenden 17-(Keto)-steroiden durch synthetischen Aufbau der Pregnan-Seitenkette (J. Fried und J. A. Edwards: Organic reactions in steroid chemistry; Vol. 2, S. 127; van Nostrand Reinhold Company, New York, 1972) hergestellt.

Es ist bekannt, daß durch partiellen mikrobiologischen Abbau der Seitenkette natürlicher Sterolide 17-(20)-De-(hydro)-23,24-di-(nor)-cholansäure-Derivate, zum Beispiel 3-(Oxo)-23,24-Di-(nor)-4,17(20)-choladien-22-säure (US-PS 4 132 408), 9α-(Hydroxy)-3-(oxo)-23,24-di-(nor)-4,17(20)-choladien-22-säure (M. G. Wovcha und Mitarbeiter, Biochim. Biophys. Acta 531 [1989], 308, EP-OS 0011235 sowie A. Jekkel und Mitarbeiter, J. Gen. Microbiol. 135 [1989], 1 727) und 3β-(Hydroxy)-23,24-di-(nor)-5,17(20)-choladien-22-säure (HU-PS 190 665) hergestellt werden können.

Die natürlichen Sterine, darunter insbesondere diejenigen pflanzlichen Ursprunges, wie das aus Sojaöl gewonnene Gemisch aus β-Sitosterin und Kampesterin, ferner die aus den Nebenprodukten der Holzverarbeitung gewinnbaren Steringemische sind in großen Mengen vorhanden und kostengünstig. Die Erfindung richtet sich daher darauf, die aus diesen Sterinen durch mikrobiologischen Abbau wirtschaftlich herstellbaren 17(20)-De-(hydro)-23,24-di-(nor)-cholansäure-Derivate in Zwischenprodukte mit Pregnanstruktur umzuwandeln und diese für die Arzneimittelsynthese einzusetzen.

Die 9α-(Hydroxy)-3-(oxo)-23,24-di-(nor)-4,17(20)-choladien-22-säure ist besonders für die Herstellung von Hydrocortison und entzündungshemmenden Corticosteroid-Arzneimitteln eine günstige Ausgangssubstanz, weil aus ihr durch Abspalten der 9α-(Hydroxy)-gruppe mit Chlorsulfonsäure 3-(Oxo)-23,24-di-(nor)-4,9(11),17(20)-cholatrien-22-säure hergestellt werden kann (GB-PS 2 199 325) und die in dieser Verbindung in der 9(11)-Stellung vorliegende Doppelbindung das Einbringen der für die Corticosteroide charakteristischen 11β-(Hydroxy)-gruppe sowie der die entzündungshemmende Wirkung steigernden Fluor- und Chlorsubstituenten in die 9α-Stellung des Sterangerüstes ermöglicht (V. van Rheenen und K. P. Shephard: J. Org. Chem. 44 [1979], 1 582; G. R. Allen, M. J. Weiss: J. Am. Chem. Soc. 81 [1959], 4 968).

Auch die in den entzündungshemmenden Corticosteroiden und den gestagen wirkenden Arzneimitteln gleichermaßen vorkommende Doppelbindung kann in die 17(20)-De-(hydro)-23,24-di-(nor)-cholansäuren eingebaut werden. So kann zum Beispiel die 3-(Oxo)-23,24-di-(nor)-4,9(11),17(20)-cholatrien-22-säure auf mikrobiolgischem Wege mittels Arthrobacter simplex zu 3-(Oxo)-23,24-di-(nor)-1,4,9(11),17(20)-cholatetraen-22-säure dehydriert werden (HU-PS 200 203).

Der Erfindung liegt die Aufgabe zugrunde, aus aus natürlichen Sterinen gewinnbaren Ausgangssubstanzen herstellbare neue Zwischenprodukte zur Herstellung von 3-(oxo)- oder 3-(hydroxy)-substituierten 3-(oxo)- oder 3-(hydroxy)-substituierten 17α-(Hydroxy)-20-(oxo)-pregnan-Derivaten, die Verwendung dieser Zwischenprodukte sowie ein einfaches Verfahren zur Herstellung der 3-(oxo)- beziehungsweise 3-(hydroxy)-substituierten 17α-(Hydroxy)-20-(oxo)-pregnan-Derivate und ein Verfahren zur Herstellung der genannten Zwischenprodukte zu schaffen.

Das Obige wurde überraschenderweise durch die Erfindung erreicht.

In eigenen Versuchen wurde nun überraschenderweise festgestellt, daß aus natürlichen Sterinen einfach gewinnbare 3-(oxo)- oder 3-(hydroxy)-substituierte 23,24-Di-(nor)-17,20-cholensäure-Derivate oder anders ausgedrückt 17(20)-De-(hydro)-23,24-di-(nor)-cholansäure-Derivate durch in Gegenwart von Salzen von Sauerstoffsäuren von Übergangsmetallen, insbesondere Ammonium-paramolybdat oder Natriumwolframat, oder mit tertiären Aminen oder Säureamiden gebildeten Komplexen von solchen Übergangsmetall-Persäure- oder Polysäuresalzen und in Pyridin als Reaktionsmedium durchgeführte Umsetzung mit Wasserstoffperoxyd selektiv zu im Schrifttum nicht beschriebenen 3-(oxo)- beziehungsweise 3-(hydroxy)-substituierten 17α,20-(Epoxy)-Derivaten umgesetzt werden und die aus diesen neuen 3-(oxo)- oder 3-(hydroxy)-substituierten 17α,20-(Epoxy)-pregnan-20-carbonsäure-Derivaten oder anders ausgedrückt 17α,20-(Epoxy)-23,24-di-(nor)-cholansäure-Derivaten gebildeten Säureazide beziehungsweise aus den letzteren herstellbaren Isocyanate sich beim Erwärmen umlagern und unter der Einwirkung von wäßriger Säure zu 3-(oxo)-oder 3-(hydroxy)-substituierten 17α-(Hydroxy)-20-(oxo)-pregnan-Derivaten zersetzen.

Gegenstand der Erfindung sind daher die neuen Zwischenprodukte 3-(Oxo)- oder 3-(hydroxy)-substituierte 17α,20-(Epoxy)-pregnan-20-carbonsäure- beziehungsweise -isocyanat-Derivate der allgemeinen Formel

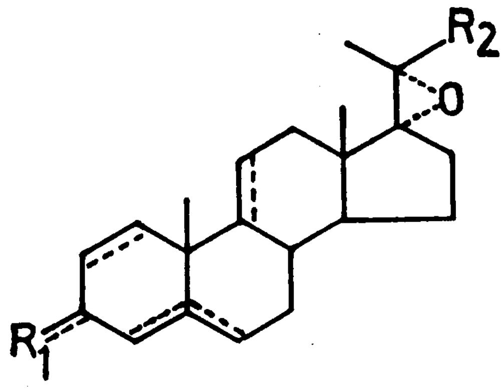

I ,

worin

R₁ für eine Hydroxy- oder Oxogruppe steht,

R₂ eine Carboxylgruppe der Formel

$$- \overset{O}{\overset{\parallel}{C}} - OH,$$

eine Carbonsäureanhydridgruppe, insbesondere der Formel

$$- \overset{O}{\overset{\parallel}{C}} - O - \overset{O}{\overset{\parallel}{C}} - O - C_{1-4}\text{-Alkyl},$$

eine Carbonsäureazidogruppe der Formel

$$- \overset{O}{\overset{\parallel}{C}} - N_3$$

oder eine Isocyanatgruppe der Formel - N = C = O bedeutet und

die gestrichelten Linien gegebenenfalls vorhandene weitere chemische Bindungen darstellen, wobei nicht gleichzeitig in den 4(5)- und 5(6)- Stellungen Doppelbindungen vorliegen können.

Durch deren Weiterverarbeitung können in einfacher Weise 3-(oxo)- oder 3-(hydroxy)-substituierte 17α-(Hydroxy)-20-(oxo)-pregnan-Derivate erhalten werden.

Bevorzugte erfindungsgemäße 3-(oxo)- oder 3-(hydroxy)-substituierte 17α,20-(Epoxy)-pregnan-20-carbonsäure- beziehungsweise -isocyanat-Derivate sind

3-(Oxo)-17α,20-(epoxy)-23,24-di-(nor)-4-cholen-22-säure,
3-(Oxo)-17α,20-(epoxy)-23,24-di-(nor)-4,9(11)-choladien-22-säure,
3-(Oxo)-17α,20-(epoxy)-23,24-di-(nor)-1,4,9(11)-cholatrien-22-säure,
3β-(Hydroxy)-17α,20-(epoxy)-23,24-di-(nor)-5-cholen-22-säure,
3-(Oxo)-17α,20-(epoxy)-23,24-di-(nor)-4-cholen-22-säureazid,
3-(Oxo)-17α,20-(epoxy)-23,24-di-(nor)-4,9(11)-choladien-22-säureazid,
3-(Oxo)-17α,20-(epoxy)-23,24-di-(nor)-1,4,9(11)-cholatrien-22-säureazid und

3β-(Hydroxy)-17α,20-(epoxy)-23,24-di-(nor)-5-cholen-22-säureazid.

Gegenstand der Erfindung ist auch die Verwendung der erfindungsgemäßen 3-(oxo)-beziehungsweise 3-(hydroxy)-substituierten 17α,20-(Epoxy)-pregnan-20-carbonsäure-beziehungsweise -isocyanat-Derivate der allgemeinen Formel I, bei welchen $R_2$ für eine Isocyanatgruppe oder Carbonsäureazidogruppe steht, zur Herstellung von 3-(oxo)- oder 3-(hydroxy)-substituierten 17α-(Hydroxy)-20-(oxo)-pregnan-Derivaten der allgemeinen Formel

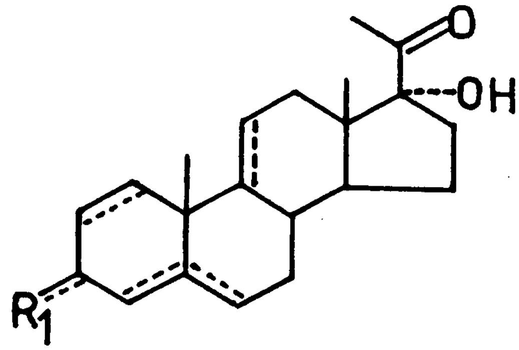

II ,

worin

$R_1$ und die gestrichelten Linien die oben angegebenen Bedeutungen haben,

durch Umsetzen mit Mineralsäuren oder organischen Säuren in wasserhaltigen organischen Lösungsmitteln.

Ferner ist Gegenstand der Erfindung ein Verfahren zur Herstellung der 3-(oxo)- oder 3-(hydroxy)-substituierten 17α-(Hydroxy)-20-(oxo)-pregnan-Derivate der allgemeinen Formel

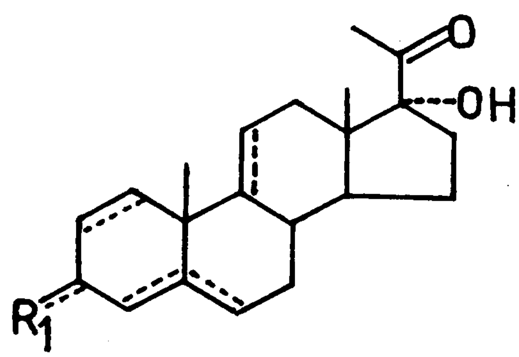

II ,

worin

$R_1$ und die gestrichelten Linien die oben angegebenen Bedeutungen haben,

welches dadurch gekennzeichnet ist, daß

a) in situ hergestellte 3-(oxo)- oder 3-(hydroxy)-substituierte 17α,20-(Epoxy)-pregnan-20-isocyanat-Derivate der allgemeinen Formel I, bei welchen $R_2$ für eine Isocyanatgruppe steht, in wasserhaltigen organischen Lösungsmitteln mit Mineralsäuren oder organischen Säuren oder solche Säuren liefernden Reagenzien umgesetzt werden.

b) 3-(oxo)- oder 3-(hydroxy)-substituierte 17α,20-(Epoxy)-pregnan-20-carbonsäureazid-Derivate oder [17α,20-(Epoxy)-23,24-di-(nor)-cholansäureazid-Derivate] der allgemeinen Formel I, bei welchen $R_2$ für eine Carbonsäureazidogruppe steht, in wasserhaltigen organischen Lösungsmitteln bei Temperaturen von mindestens 40°C mit Mineralsäuren oder organischen Säuren oder solche Säuren liefernden Reagenzien umgesetzt werden oder

c) aus 3-(oxo)- oder 3-(hydroxy)-substituierten 17α,20-(Epoxy)-pregnan-20-carbonsäure-Derivaten oder [17α,20-(Epoxy)-23,24-di-(nor)-cholansäure-Derivaten] der allgemeinen Formel I, bei welchen $R_2$ für eine Carboxylgruppe steht, hinsichtlich der Carboxylfunktion reaktionsfähige Derivate hergestellt werden, diese mit Azidionen enthaltenden Salzen zu 3-(oxo)- oder 3-(hydroxy)-substituierten 17α,20-(Epoxy)-pregnan-20-carbonsäureazid-Derivaten [17α,20-(Epoxy)-23,24-di-(nor)-cholansäureazid-Derivaten] der allgemeinen Formel I, bei welchen $R_2$ für eine Carbonsäureazidogruppe steht, umgesetzt werden und die letzteren in wasserhaltigen organischen Lösungsmitteln bei Temperaturen von mindestens 40° C mit

Mineralsäuren oder organischen Säuren umgesetzt werden
oder

d) 3-(oxo)- oder 3-(hydroxy)-substituierte 23,24-Di-(nor)-17(20)-cholensäure-Derivate oder oder anders ausgedrückt 17(20)-Dehydro-23,24-di-nor)-cholansäure-Derivate der allgemeinen Formel

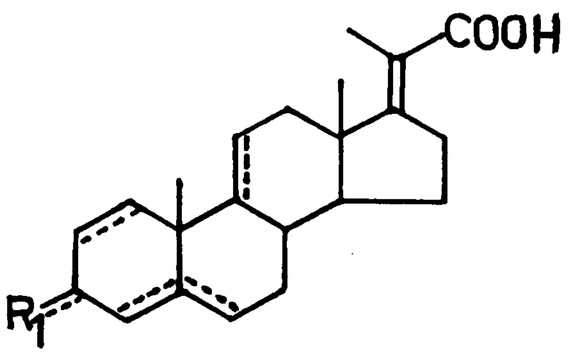

III ,

worin

$R_1$ und die gestrichelten Linien die oben angegebenen Bedeutungen haben,

in wasserhaltigen organischen Lösungsmitteln in Gegenwart von Salzen von Sauerstoffsäuren oder Persäuren oder Polysäuren von Übergangsmetallen oder mit tertiären Aminen oder Säureamiden gebildeten Komplexen von solchen Übergangsmetall-Persäure- oder -Polysäuresalzen mit Wasserstoffperoxyd umgesetzt werden, aus den erhaltenen 3-(oxo)- oder 3-(hydroxy)-substituierten 17α,20-(Epoxy)-pregnan-20-carbonsäure-Derivaten [17α,20-(Epoxy)-23,24-di-(nor)-cholansäure-Derivaten der allgemeinen Formel I, bei welchen $R_2$ für eine Carboxylgruppe steht, reaktionsfähige Derivate hergestellt werden, diese mit Azidionen enthaltenden Salzen zu 3-(oxo)- oder 3-(hydroxy)-substituierten 17α,20-(Epoxy)-pregnan-20-carbonsäureazid-Derivaten [17α,20-(Epoxy)-23,24-di-(nor)-cholansäureazid-Derivate] der allgemeinen Formel I, bei welchen $R_2$ für eine Carbonsäureazidogruppe steht, umgesetzt werden und die letzteren in wasserhaltigen organischen Lösungsmitteln bei Temperaturen von mindestens 40 °C mit Mineralsäuren oder organischen Säuren umgesetzt werden.

Vorzugsweise wird die Umsetzung der 3-(oxo)- oder 3-(hydroxy)-substituierten 17α,20-(Epoxy)-pregnan-20-isocyanat-Derivate der allgemeinen Formel I der Variante a) des obigen erfindungsgemäßen Verfahrens in aliphatischen Alkoholen mit 1 bis 5 Kohlenstoffatom(en) oder N,N-Di-(methyl)-formamid mit wäßriger Essigsäure als organischer Säure durchgeführt. Zweckmäßig werden bei dieser Umsetzung Temperaturen von mindestens 40 °C, insbesondere von 40 °C bis zur Siedetemperatur des verwendeten Lösungsmittelmediums, angewandt. Als Essigsäure wird vorzugsweise eine 40 bis 60 gew. -%-ige, insbesondere 50 gew.-%-ige, eingesetzt.

Die 3-(oxo)- oder 3-(hydroxy)-substituierten 17α,20-(Epoxy)-pregnan-20-isocyanat-Derivate der allgemeinen Formel I können aus den entsprechenden 3-(oxo)- oder 3-(hydroxy)-substituierten 17 ,20-(Epoxy)-pregnan-20-carbonsäureazid-Derivaten der allgemeinen Formel I durch Umlagerung in der Wäreme, im allgemeinen bei Temperaturen über 40 °C, erhalten worden sein. Bei dieser Umsetzung ist das Isocyanat im Reaktionsgemisch mittels Spektroskopie nachweisbar, aber nicht isolierbar.

Vorzugsweise wird die Umsetzung der 3-(oxo)- oder 3-(hydroxy)-substituierten 17α,20-(Epoxy)-pregnan-20-carbonsäureazid-Derivate der allgemeinen Formel I der Variante b) des obigen erfindungsgemäßen Verfahrens in aliphatischen Alkoholen mit 1 bis 5 Kohlenstoffatom(en) oder in Dimethylformamid mit wäßriger Essigsäure als organischer Säure bei Temperaturen von 40 °C bis zur Siedetemperatur des verwendeten Lösungsmittelmediums durchgeführt. Als Essigsäure wird vorzugsweise eine 10 bis 40 gew.-%-ige, insbesondere 10 bis 25 gew.-%-ige, eingesetzt.

Die Herstellung der genannten reaktionsfähigen Derivate bei den Varianten c) und d) des obigen erfindungsgemäßen Verfahrens kann in an sich bekannter Weise durchgeführt werden.

Es ist bevorzugt, bei der Variante c) des erfindungsgemäßen Verfahrens aus den 3-(oxo)- oder 3-(hydroxy)-substituierten 17α,20-(Epoxy)-pregnan-20-carbonsäure-Derivaten der allgemeinen Formel I als reaktionsfähige Derivate gemischte Anhydride herzustellen, diese, gegebenenfalls ohne zwischenzeitliches Isolieren, mit Alkalimetallaziden als Azidionen enthaltenden Salzen umzusetzen und die erhaltenen 3-(oxo)- oder 3-(hydroxy)-substituierte 17α,20-(Epoxy)-pregnan-20-carbonsäureazid-Derivate der allgemeinen Formel I, gegebenenfalls ohne zwischenzeitliches Isolieren, zu den entsprechenden 3-(oxo)- oder 3-(hydroxy)-substituierten 17α-(Hydroxy)-20-(oxo)-pregnan-Derivaten der allgemeinen Formel II umzusetzen.

Von den als reaktionsfähige Derivate der 3-(oxo)- oder 3-(hydroxy)-substitierten 17α,20-(Epoxy)-pregnan-20-carbonsäure-Derivate der allgemeinen Formel I bevorzugten gemischten Anhydriden ist die Verwendung von solchen der allgemeinen Formel I, bei welchen $R_2$ für eine Carbonsäureanhydridgruppe der Formel

$$-\overset{\overset{\displaystyle O}{\|}}{C}-O-\overset{\overset{\displaystyle O}{\|}}{C}-O-C_{1-4}\text{-Alkyl}$$

steht, besonders bevorzugt. Sie können mit $C_1$-$C_4$-Alkylestern der Chlorameisensäure, insbesondere Chlorameisensäuremethyl-, -äthyl- oder -isobutylester, hergestellt werden. Die Umsetzung wird zweckmäßig in Gegenwart von tertiären Aminbasen, insbesondere Triäthylamin, bei Temperaturen von -20 bis +20 °C durchgeführt.

Vorzugsweise werden die Zwischenprodukte darstellenden gemischten Anhydride der 3-(oxo)- oder 3-(hydroxy)-substituierten 17α,20-(Epoxy)-pregnan-20-carbonsäure-Derivate der allgemeinen Formel I bei der Variante c) oder d) des erfindungsgemäßen Verfahrens ohne zwischenzeitliches Isolieren unmittelbar mit den Azidanionen enthaltenden Salzen, insbesondere Alkalimetallaziden, ganz besonders Nätriumazid zu den 3-(oxo)- oder 3-(hydroxy)-substituierten 17α,20-(Epox)-pregnan-20-carbonsäureazid-Derivaten der allgemeinen Formel I umgesetzt. Als Temperaturen werden zweckmäßig höchstens 45 °C angewandt.

Die im erfindungsgemäßen Verfahren als Zwischenprodukte dienenden 3-(oxo)- oder 3-(hydroxy)-substituierten 17α, 20-( Epoxy)-pregnan-20-carbonsäureazid-Derivate der allgemeinen Formel I können gegebenenfalls aus dem Reaktionsgemisch abgetrennt werden. Bevorzugt ist es jedoch, diese ohne zwischenzeitliches Isolieren weiter umzusetzen.

Nach einer bevorzugten Ausführungsform der Variante d) des obigen erfindungsgemäßen Verfahrens werden bei der Umsetzung der 3-(oxo)- oder 3-(hydroxy)-substituierten 23,24-Di-(nor)-17(20)-cholensäure-Derivate der allgemeinen Formel III zu den 3-(oxo)- oder 3-(hydroxy)-substituierten 17α,20-(Epoxy)-pregnan-20-carbonsäure-Derivaten der allgemeinen Formel I als organische Lösungsmittel Stickstoff enthaltende, insbesondere Pyridin, N,N-Di-(methyl)-formamid oder Hexamethylphosphorsäuretriamid, verwendet und die Umsetzung wird bei Temperaturen von 0 bis 100 °C durchgeführt. Als Wasserstoffperoxyd wird vorzugsweise 25 bis 35 gew. -%-iges, insbesondere 30 gew.-%-iges, wäßriges eingesetzt. Als Temperaturen sind 20 bis 100 °C bevorzugt.

Vorzugsweise werden als Salze von Sauerstoffsäuren oder Persäuren oder Polysäuren von Übergangsmetallen $MoO_4^{--}$- oder $WO_4^{--}$- Ionen oder deren peroxydierte Formen enthaltende Salze, insbesondere Ammoniumparamolybdat oder Natriumwolframat, wie der Formel $(NH_4)_6MO_7O_{24}$ x 4 $H_2O$ bzw. $Na_2WO_4$ , verwendet. Im Falle der Verwendung von Natriumwolframat als Katalysator werden besonders bevorzugt als Reaktionstemperatur 40 bis 80 °C und im Falle der Verwendung von Ammoniummolybdat 20 bis 50 °C angewandt. Die bei der Variante d) des obigen erfindungsgemäßen Verfahrens verwendbaren Persäuresalze von Übergangsmetallen beziehungsweise Persäureionen enthaltenden Oxosäuresalze von Übergangsmetallen, wie Perwolframat- oder Permolybdationen enthaltende Salze, können in situ im Reaktionsgemisch hergestellt worden sein. Die Herstellung von $Na_2WO_6$ kann zum Beispiel nach V.A. Lunenok-Burmakina und Mitarbeitern {Zh. Fiz. Khim. 43 [1969] (11), 2 723} erfolgen. Beispiele für ebenfalls als aktive Reagenzien zur Epoxydbildung bei der Variante d) des obigen erfindungsgemäßen Verfahrens verwendbare Komplexe von Übergangsmetall-Persäuresalzen sind mit verschiedenen Stickstoff enthaltenden organischen Verbindungen, zum Beispiel mit 8-(Hydroxy)-chinolin, α,α -Di-(pyridyl)- oder Hexamethylphosphorsäuretriamid, gebildete Komplexe von Permolybdaten und Perwolframaten. Als spezielle Beispiele für derartige Komplexe seien $H_2MoO_6$ x OP $(NMe_2)_3$ (DE-PS 1 815 998) und $H_2WO_6$ x α,α-Dipyridyl (R. G. Beiles und E. M. Beiles: Zh. Neorg. Khim. 12 [1967] (5), 1 399 erwähnt. In diesem Fall wird die Epoxydbildung zweckmäßig in wasserhaltigen, mit Wasser mischbaren organischen Lösungsmitteln, zum Beispiel tert.-Butanol, durchgeführt.

Die bei der genannten Umsetzung erhaltenen 3-(oxo)- oder 3-(hydroxy)-substituierten 17α,20-(Epoxy)-pregnan-20-carbonsäure-Derivate können in an sich bekannter Weise aus dem Reaktionsgemisch abgetrennt werden.

Die Zwischenprodukte 3-(oxo)- oder 3-(hydroxy)-substituierte 17α,20-(Epoxy)-pregnan-20-carbonsäureazid-Derivate der allgemeinen Formel I können auch aus den 3-(oxo)- oder 3-(hydroxy)-substituierten 17α,20-(Epoxy)-pregnan-20-carbonsäurehydrazid-Derivaten oder anders ausgedrückt 17α,20-(Epoxy)-23,24-di-(nor)-cholansäurehydrazid-Derivaten durch Nitrosieren oder aus den 3-(oxo)- oder 3-

(hydroxy)-substituierten 17α,20-(Epoxy)-pregnan-20-carbonsäurechlorid-Derivaten oder anders ausgedrückt 17α,20-(Epoxy)-23,24-di-(nor)-cholansäurechlorid-Derivaten mit Alkalimetallaziden, vorzugsweise Natriumazid, hergestellt werden.

Gegenstand der Erfindung ist daher auch ein Verfahren zur Herstellung der erfindungsgemäßen 3-(oxo)- oder 3-(hydroxy)-substituierten 17α,20-(Epoxy)-pregnan-20-carbonsäure- beziehungsweise -isocyanat-Derivate der allgemeinen Formel I, welches dadurch gekennzeichnet ist, daß

a) 3-(oxo)- oder 3-(hydroxy)-substituierte 23,24-Di-(nor)-17(20)-cholensäure-Derivate oder anders ausgedrückt 17(20)-Dehydro-23,24-di-(nor)-cholansäure-Derivate der allgemeinen Formel III in wasserhaltigen organischen Lösungsmitteln in Gegenwart von Salzen von Sauerstoffsäuren oder Persäuren oder Polysäuren von Übergangsmetallen oder mit tertiären Aminen oder Säureamiden gebildeten Komplexen von solchen Übergangsmetall-Persäure- oder Polysäuresalzen mit Wasserstoffperoxyd umgesetzt werden sowie gegebenenfalls aus den erhaltenen 3-(oxo)- oder 3-(hydroxy)-substituierten 17α,20-(Epoxy)-pregnan-20-carbonsäure-Derivaten 17α,20-(Epoxy)-23,24-di-(nor)-cholansäure-Derivaten der allgemeinen Formel I, bei welchen $R_2$ für eine Carboxylgruppe steht, reaktionsfähige Derivate hergestellt werden und gegebenenfalls diese mit Azidionen enthaltenden Salzen zu 3-(oxo)- oder 3-(hydroxy)-substituierten 17α,20-(Epoxy)-pregnan-20-carbonsäureazid-Derivaten [17α,20-(Epoxy)-23,24-di-(nor)-cholansäureazid-Derivaten] der allgemeinen Formel I, bei welchen $R_2$ für eine Carbonsäureazidgruppe steht, umgesetzt werden oder

b) 3-(oxo)- oder 3-(hydroxy)-substituierte 17α,20-(Epoxy)-pregnan-20-carbonsäurehydrazid-Derivate (allgemeine Formel I mit der Abwandlung, daß $R_2$ eine Carbonsäurehydrazidogruppe der Formel

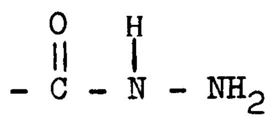

bedeutet) zu 17α,20-(Epoxy)-pregnan-20-carbonsäureazid-Derivaten der allgemeinen Formel I, bei welchen $R_2$ für eine Carbonsäureazidogruppe steht, nitrosiert werden oder

c) 3-(oxo)- oder 3-(hydroxy)-substituierte 17α,20-(Epoxy)-pregnan-20-carbonsäurehalogenid-Derivate, insbesondere 17α,20-(Epoxy)-pregnan-20-carbonsäurechlorid-Derivate (allgemeine Formel I mit der Abwandlung, daß

$R_2$ eine Carbonsäurehalogenidgruppe der allgemeinen Formel

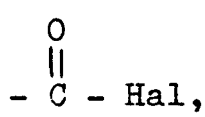

insbesondere Carbonsäurechloridgruppe der Formel

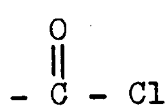

bedeutet)

mit Alkalimetallaziden, insbesondere Natriumazid, zu 17α,20-(Epoxy)-pregnan-20-carbonsäureazid-Derivaten der allgemeinen Formel I, bei welchen $R_2$ für eine Carbonsäureazidogruppe steht, umgesetzt werden

oder

d) 3-(oxo)- oder 3-(hydroxy)-substituierte 17α,20-(Epoxy)-pregnan-20-carbonsäureazid-Derivate der allgemeinen Formel I, bei welchen $R_2$ für eine Carbonsäureazidogruppe steht, in der Wärme, im allgemeinen bei Temperaturen von mindestens 40° C, zu 3-(oxo)- oder 3-(hydroxy)-substituierten 17α,20-(Epoxy)-pregnan-20-isocyanat-Derivaten umgelagert werden.

Für die Variante a) des letztgenannten erfindungsgemäßen Verfahrens gelten die Bevorzugungen für die Varianten d) und c) des weiter oben beschriebenen erfindungsgemäßen Verfahrens.

Die Struktur der 3-(oxo)- oder 3-(hydroxy)-substituierten 17α-(Hydroxy)-20-(oxo)-pregnan-Derivate der allgemeinen Formel II und der isolierten Zwischenprodukte der allgemeinen Formel I wurde durch UV-, IR-, $^{1}H$- und $^{13}C$-NMR- sowie durch Massenspektroskopie sichergestellt.

Die durch das erstgenannte erfindungsgemäße Verfahren erhaltenen bekannten 3-(oxo)- oder 3-(hydroxy)-substituierten 17α-(Hydroxy)-20-(oxo)-pregnan-Derivate der allgemeinen Formel II haben therapeutisch wertvolle Eigenschaften und können auch als Zwischenprodukte zur Herstellung von anderen therapeutisch wertvollen Substanzen dienen.

Die Erfindung wird an Hand der folgenden Beispiele näher erläutert.

**Beispiel 1**

Herstellung von 17α-Hydroxy-4-pregnen-3,20-dion aus 3-Oxo-23,24-dinor-4,17(20)-choladien-22-säure

a) 3-Oxo-17α,20-epoxy-23,24-dinor-4-cholen-22-säure

Zu einem Gemisch aus 343 mg (1 mMol) 3-Oxo-23,24-dinor-4,17(20)-choladien-22-säure, 5 ml Pyridin und 0,4 ml 0,5 M $Na_2WO_4$-Lösung werden bei 60 °C 0,4 ml 30 %ige Wasserstoffperoxydlösung gegeben. Nach 15 Minuten wird das Reaktionsgemisch abgekühlt, in 200 ml kalte 0,25 %ige $Na_2SO_3$-Lösung gegossen, und der pH-Wert der Lösung wird mit 6M HCl auf 3 gestellt. Der ausgefallene Niederschlag wird abfiltriert, mit Wasser gewaschen und Zuerst an der Luft, dann über $P_2O_5$ getrocknet. Man erhält 340 mg Produkt, das aus Aceton umkristallisiert wird. Fp.: 190-195 °C.

| Elementaranalyse für $C_{22}H_{30}O_4$ (M = 358,48): | | |
|---|---|---|
| berechnet, %: | C 73,72 | H 8,44 |
| gefunden, %: | C 73,5 | H 8,51 |

UV (Äthanol): $\lambda_{max}$: 241 nm;
IR (KBr, $cm^{-1}$): $\nu_{OH}$ 3200 (breit), $\nu_{C=O}$ 1725, 1645; $\nu_{C=C}$ 1615;
$^{1}H$-NMR (DMSO-$d_6$, δ): 5,65 (d, J = 1,7 Hz, 1 H-4), 1,51 (s, 3 H-21), 1,15 (s, 3 H-19), 0,90 (s, 3 H-18).

b) 3-Oxo-17α,20-epoxy-23,24-dinor-4-cholen-22-säureazid

Zu einem Gemisch aus 195 mg (0,54 mMol) 3-Oxo-17α,20-epoxy-23,24-dinor-4-cholen-22-säure, 5 ml Dichlormethan und 85 µl Triäthylamin werden bei -20 °C 77 µl (0,6 mMol) Chlorameisensäureisobutylester gegeben. Die Lösung wird eine Stunde lang gerührt, wobei die Temperatur allmählich auf 15 °C ansteigt. Zu der auf diese Weise erhaltenen Lösung des gemischten Anhydrids wird die Lösung von 60 mg Natriumazid und 5 mg Tetrabutylammoniumbromid in 1 ml Wasser gegeben. Das Gemisch wird bei 20 °C eine halbe Stunde lang gerührt und anschließend mit Dichloräthan und Wasser verdünnt. Die organische Phase wird mit Wasser, dann mit verdünnter Essigsäure und anschließend wieder mit Wasser gewaschen, über Natriumsulfat getrocknet und im Vakuum eingeengt. Das Lösungsmittel wird gegen Methanol ausgetauscht, aus dem das Produkt kristallisiert. Fp.: 69-70 °C.

| Elementaranalyse für $C_{22}H_{29}N_3O_3$ (M = 383,50): | | | |
|---|---|---|---|
| berechnet, %: | C 68,90 | H 7,62 | N 10,96 |
| gefunden, %: | C 68,32 | H 7,62 | N 10,38 |

UV (Äthanol): $\lambda_{max}$: 239 nm;
IR (KBr, $cm^{-1}$): $\nu_{Azid}$ 2150, $\nu_{C=O}$ 1715, 1670; $\nu_{C=C}$ 1610;
$^{1}H$-NMR (DMSO-$d_6$, δ): 5,73 (d, J = 1,7 Hz, 1 H-4), 1,63 (s, 3 H-21), 1,18 (s, 3 H-19), 0,96 (s, 3 H-18).

c) 17α-Hydroxy-4-pregnen-3,20-dion

Zu der Lösung von 153 mg (0,4 mMol) 3-Oxo-17α,20-epoxy-23,24-dinor-4-cholen-22-säureazid in 200 µl Chloroform wird das heiße Gemisch von 3 ml Dimethylformamid und 3 ml 20 %iger Essigsäure gegeben. Das Reaktionsgemisch wird 3 Stunden lang am Rückfluß gekocht und dann im Vakuum eingeengt. Das

Rohprodukt wird an einer mit Silikagel gefüllten Säule chromatographisch gereinigt. Durch Eluieren mit einem 92:8-Gemisch aus Toluol und Aceton erhält man das reine Produkt, das bei 218-221 °C schmilzt (220-222 °C; H. J. Ringold et al.: J. Am. Chem. Soc. 78, 816 /1956/).

| Elementaranalyse für $C_{21}H_{30}O_3$ (M = 330,47): | | |
|---|---|---|
| berechnet, %:<br>gefunden, %: | C 76,32<br>C 76,18 | H 9,15<br>H 9,28 |

$[\alpha]_D$: +94° (c = 1, Chloroform) (gemäß der oben zitierten Literatur +95°).
UV (Äthanol): $\lambda_{max}$: 240 nm;
IR (KBr, $cm^{-1}$): $\nu_{OH}$ 3310 (breit), $\nu_{C=O}$ 1705, 1660; $\nu_{C=C}$ 1610;
$^1$H-NMR (DMSO-$d_6$, δ): 5,74 (d, J = 1,7 Hz, 1 H-4), 2,28 (s, 3 H-21), 1,19 (s, 3 H-19), 0,76 (s, 3 H-18).

**Beispiel 2**

Herstellung von 17α-Hydroxy-4,9(11)-pregnadien-3,20-dion aus 3-Oxo-23,24-dinor-4,9(11),17(20)-cholatrien-22-säure

a) 3-Oxo-17α,20-epoxy-23,24-dinor-4,9(11)-choladien-22-säure

Zu der Lösung von 10,22 g (30 mMol) 3-Oxo-23,24-dinor-4,9(11),17(20)-cholatrien-22-säure in 150 ml Pyridin werden 10 ml 0,5 M $Na_2WO_4$-Lösung gegeben und dann bei 60 °C unter Rühren 10 ml 30 %ige Wasserstoffperoxyd-Lösung zugetropft. Nach 15 Minuten wird das Reaktionsgemisch abgekühlt und in 2 Liter 0,25 %ige Natriumsulfitlösung eingegossen. Das wässrige Gemisch wird mit 6 M HCl auf pH 3 angesäuert und dann bei 5 °C 2 Stunden lang stehengelassen. Das ausgefallene Produkt wird abfiltriert, mit verdünnter Salzsäure und dann mit Wasser gewaschen und über Phosphorpentoxyd getrocknet. Man erhält 10,39 g 3-Oxo-17α,20-epoxy-23,24-dinor-4,9(11)-choladien-22-säure, die aus Aceton umkristallisiert wird. Fp.: 202-205 °C.

| Elementaranalyse für $C_{22}H_{28}O_4$ (M = 356,47): | | |
|---|---|---|
| berechnet, %:<br>gefunden, %: | C 74,13<br>C 74,17 | H 7,92<br>H 7,98 |

UV (Äthanol): $\lambda_{max}$: 240 nm;
IR (KBr, $cm^{-1}$): $\nu_{OH}$ 3320 (breit), $\nu_{C=O}$ 1750, 1665; $\nu_{C=C}$ 1615;
$^1$H-NMR (DMSO-$d_6$, δ): 5,68 (d, J = 1,7 Hz, 1 H-4), 5,46 (dd, J = 5,8 und 1,8 Hz, 1 H-11), 1,5 (s, 3 H-21), 1,32 (s, 3 H-19), 0,85 (s, 3 H-18).

b) 3-Oxo-17α,20-epoxy-23,24-dinor-4,9(11)-choladien-22-säureazid

5,35 g (15 mMol) 3-Oxo-17α,20-epoxy-23,24-dinor-4,9(11)-choladien-22-säure werden in 100 ml Dichlormethan und 2,1 ml Triäthylamin gelöst. Zu der erhaltenen Lösung werden bei -20 °C unter Rühren 1,55 ml Chlorameisensäureäthylester tropfenweise zugegeben, dann läßt man die Temperatur innerhalb von 30 Minuten auf 15 °C ansteigen. Zu der Lösung des gemischten Anhydrids wird die Lösung von 1,46 g (22,5 mMol) Natriumazid in 15 ml Wasser gegeben. Das zweiphasige Gemisch wird bei 20 °C eine halbe Stunde lang gerührt, dann mit Wasser verdünnt und mit Dichlormethan extrahiert. Der Extrakt wird erst mit 5 %iger $NaHCO_3$-Lösung, dann mit Wasser gewaschen, über wasserfreiem Natriumsulfat getrocknet und im Vakuum vorsichtig eingedampft. Das Rohprodukt wird aus einem Gemisch von Dichlormethan und Aceton umkristallisiert. Man erhält 4,70 g (82 %) 3-Oxo-17α,20-epoxy-23,24-dinor-4,9(11)-choladien-22-säureazid. Schmelzverhalten: die Kristalle verlieren bei 68-69 °C Stickstoff und schmelzen bei 165-170 °C.

| Elementaranalyse für $C_{22}H_{27}N_3O_3$ (M = 381,48): | | | |
|---|---|---|---|
| berechnet, %: | C 69,27 | H 7,13 | N 11,02 |
| gefunden, %: | C 69,52 | H 7,41 | N 10,90 |

UV (Äthanol): $\lambda_{max}$: 238 nm;
IR (KBr, $cm^{-1}$): $\nu_{Azid}$ 2140 , $\nu_{C=O}$ 1705, 1670; $\nu_{C=C}$ 1615;
$^1$H-NHR (DMSO-$d_6$, $\delta$): 5,76 (d, J = 1,7 Hz, 1 H-4), 5,49 (dd, J = 5,8 und 1,8 Hz, 1 H-11), 1,63 (s, 3 H-21), 1,35 (s, 3 H-19), 0,93 (s, 3 H-18).

c) 17$\alpha$-Hydroxy-4,9(11)-pregnadien-3,20-dion

Zu der Lösung von 572 mg (1,5 mMol) 3-Oxo-17$\alpha$,20-epoxy-23,24-dinor-4,9(11)-choladien-22-säureazid in 0,5 ml Chloroform wird ein heißes Gemisch aus 30 ml Wasser, 6 ml Eisessig, 15 ml t-Amylalkohol und 10 ml t-Butylalkohol gegeben. Das Reaktionsgemisch wird 4 Stunden lang am Rückfluß gekocht und dann im Vakuum eingeengt. Das Rohprodukt (444 mg) wird an einer Silikagelsäule chromatographisch gereinigt (Elutionsmittel Toluol:Aceton = 92:8). Fp.: 211-215 °C (212-217 °C nach H. Reimann et al.: J. Org. Chem. 26, 866 /1961/).

| Elementaranalyse für $C_{21}H_{28}O_3$ (M = 328,45): | | |
|---|---|---|
| berechnet, %: | C 76,79 | H 8,59 |
| gefunden, %: | C 76,79 | H 8,80 |

$[\alpha]_D$: +69,6° (c = 0,8; Chloroform) (gemäß der oben zitierten Literatur +69°).
UV (Äthanol): $\lambda_{max}$: 240 nm;
IR (KBr, $cm^{-1}$): $\nu_{OH}$ 3355 (breit), $\nu_{C=O}$ 1705, 1660; $\nu_{C=C}$ 1610;
$^1$H-NMR (DMSO-$d_6$, $\delta$): 5,77 (d, J = 1,7 Hz, 1 H-4), 5,56 (dd, J = 5,8 und 1,8 Hz, 1 H-11) 2,30 (s, 3 H-21), 1,34 (s, 3 H-19), 0,72 (s, 3 H-18).

**Beispiel 3**

Herstellung von 17$\alpha$-Hydroxy-4,9(11)-pregnadien-3,20-dion aus 3-Oxo-23,24-dinor-4,9(11),17(20)-cholatrien-22-säure

a) 3-Oxo-17$\alpha$,20-epoxy-23,24-dinor-4,9(11)-choladien-22-säure

Zu der Lösung von 341 mg (1 mMol) 3-Oxo-23,24-dinor-4,9(11),17(20)-cholatrien-22-säure in 5 ml Pyridin wird die Lösung von 17,3 mg $(NH_4)_6Mo_7O_{24}x4H_2O$ in 0,5 ml Wasser gegeben. Dann werden bei 30 °C 0,5 ml 30 %ige Wasserstoffperoxyd-Lösung unter Rühren zugetropft. Nach 30 Minuten wird das Reaktionsgemisch abgekühlt und in 100 ml 2 %ige Salzsäure eingegossen. Das ausgefallene Produkt wird abfiltriert, zuerst mit verdünnter Salzsäure, dann mit Wasser gewaschen und über Phosphorpentoxyd getrocknet. Man erhält 340 mg 3-Oxo-17$\alpha$,20-epoxy-23,24-dinor-4,9(11)-choladien-22-säure, die chromatographisch und spektroskopisch mit dem Produkt gemäß Beispiel 2a) identisch ist.

b) 3-Oxo-17$\alpha$,20-epoxy-23,24-dinor-4,9(11)-choladien-22-säure

Zu der Lösung von 1022 mg (3 mMol) 3-Oxo-23,24-dinor-4,9(11),17(20)-cholatrien-22-säure in 50 ml tert.-Butanol werden bei 30 °C in kleinen Portionen 1866 mg (5 mMol) $H_2MoO_6xOP(NMe_2)_3$ gegeben (hergestellt nach DE-OS 1 815 998). Das Gemisch wird 45 Minuten lang gerührt und dann in 45 ml kaltes Wasser gegossen. Nach Ansäuern der Lösung auf pH 3 wird die Festsubstanz abfiltriert, mit Wasser gewaschen und über Phosphorpentoxyd getrocknet. Man erhält 985 mg (92 %) Produkt, das chromatographisch und spektroskopisch mit dem Produkt gemäß Beispiel 2a) identisch ist.

c) 17$\alpha$-Hydroxy-4,9(11)-pregnadien-3,20-dion

Zu einem Gemisch aus 3,57 g (10 mMol) 3-Oxo-17$\alpha$,20-epoxy-23,24-dinor-4,9(11)-choladien-22-säure,

70 ml Dichlormethan und 1,4 ml Triäthylamin werden bei -30 °C 1,3 ml Chlorameisensäureisobutylester gegeben. Man läßt die Temperatur innerhalb von 30 Minuten auf 5 °C ansteigen und setzt dann 1 g Natriumazid und 30 mg Tetrabutylammoniumbromid zu. Das Reaktionsgemisch wird bei 20 °C eine halbe Stunde lang gerührt und dann mit Wasser und Dichlormethan verdünnt. Die organische Phase wird abgetrennt und zuerst mit Wasser, dann mit verdünnter Essigsäure gewaschen und anschließend im Vakuum bis zur beginnenden Kristallisation vorsichtig eingeengt. Dann wird das Reaktionsgemisch mit einem Gemisch aus 100 ml Äthanol, 50 ml Methanol und 100 ml 10 %iger Essigsäure versetzt und 3 Stunden lang am Rückfluß gekocht. Das Gemisch wird im Vakuum eingedampft, der Rückstand in Dichlormethan gelöst, die Lösung über wasserfreiem Natriumsulfat getrocknet und schließlich eingedampft. Man erhält 3,1 g Produkt, das spektroskopisch und chromatographisch mit dem Produkt gemäß Beispiel 2c) identisch ist.

**Beispiel 4**

3-Oxo-17α,20-epoxy-23,24-dinor-4,9(11)-choladien-22-kohlensäureisobutylesteranhydrid

Zu einem Gemisch aus 357 mg (1 mMol) 3-Oxo-17α,20-epoxy-23,24-dinor-4,9(11)-choladien-22-säure, 10 ml Dichlormethan und 150 µl Triäthylamin werden bei -20 °C 138 µl Chlorameisensäureisobutylester gegeben. Man läßt die Temperatur des Gemisches innerhalb von 60 Minuten auf 20 °C ansteigen. Dann wird das Gemisch mit Wasser und Dichlormethan verdünnt. Die Dichlormethanphase wird zuerst mit Wasser, dann mit verdünnter $NaHCO_3$-Lösung, anschließend mit verdünnter Essigsäure und schließlich wieder mit Wasser gewaschen und nach dem Trocknen im Vakuum vorsichtig eingedampft. Man erhält 421 mg eines farblosen Öls.

IR (Film, $cm^{-1}$): $\nu_{C=O}$ 1805, 1675; $\nu_{C=C}$ 1610;

$^1$H-NMR ($CDCl_3$, δ): 5,75 (d, J = 1,7 Hz, 1 H-4), 5,48 (dd, J = 5,8 und 1,8 Hz, 1 H-11), 4,07 (d, 2H, O-$CH_2$), 1,71 (s, 3 H-21), 1,37 (s, 3 H-19), 1,00 (d, 6H, Isopropyl), 0,69 (s, 3 H-18).

$^{13}$C-NMR: 198,8 (C-3), 123,7 (C-4), 169,5 (C-5), 36,5 (C-8), 144,1 (C-9), 40,6 (C-10), 117,7 (C-11), 41,1 (C-13), 49,8 (C-14) 24,2 (C-15), 76,6 (C-17), 13,8 (C-18), 25,8 (C-19), 61,8 (C-20), 15,3 (C-21), 166,0 (C-22) und weitere 6 $CH_2$-Signale: 34,9, 33,9, 33,4, 32,4, 31,5, 28,8 und Isobutoxycarbonyl: 148,6, 75,6, 27,3, 18,4.

**Beispiel 5**

a) 3-Oxo-17α,20-epoxy-4,9(11)-pregnadienyl-20-isocyanat

76 mg (0,2 mMol) 3-Oxo-17α,20-epoxy-23,24-dinor-4,9(11)-choladien-22-säureazid und 500 µl Dideuterotetrachloräthan werden für 3 Minuten auf 100 °C erwärmt und dann auf 10 °C abgekühlt. Die Lösung hat folgende spektroskopische Daten:

IR (Film, $cm^{-1}$): $\nu_{NCO}$ 2260; $\nu_{C=O}$ 1610;

$^1$H-NMR ($CDCl_3$, δ): 5,64 (d, J = 1,7 Hz, 1 H-4), 5,37 (dd, J = 5,8 und 1,8 Hz, 1 H-11), 1,61 (s, 3 H-21), 1,25 (s, 3 H-19), 0,78 (s, 3H-18).

$^{13}$C-NMR: 198,8 (C-3), 123,7 (C-4), 169,5 (C-5), 36,6 (C-8), 144,2 (C-9), 40,7 (C-10), 117,7 (C-11), 39,9 (C-13), 50,4 (C-14), 23,9 (C-15), 78,6 (C-17), 14,4 (C-18), 25,9 (C-19), 70,8 (C-20), 21,7 (C-21), 124,2 (Isocyanat) und weitere 6 $CH_2$-Signale: 34,3, 34,0, 33,4, 32,5, 31,6, 30,4.

b) 17α-Hydroxy-4,9(11)-pregnadien-3,20-di-on

Das 3-Oxo-17α,20-epoxy-4,9(11)-pregnadienyl-20-isocyanat gemäß Beispiel 5a) wird in Tetrachloräthan gelöst und die Lösung in die kochende Lösung von 10 ml 50 %iger Essigsäure in 10 ml Isopropanol gegossen. Das Gemisch wird 3 Stunden lang gekocht, nach dem Abkühlen eingedampft und der Rückstand auf die im Beispiel 2c) beschriebene Weise gereinigt. Das Produkt ist chromatographisch und spektroskopisch mit dem Produkt gemäß Beispiel 2c) identisch.

**Beispiel 6**

Herstellung von 17α-Hydroxy-1,4,9(11)-pregnatrien-3,20-dion aus 3-Oxo-23,24-dinor-1,4,9(11),17(20)-cholatetraen-22-säure

a) 3-Oxo-17α,20-epoxy-23,24-dinor-1,4,9(11)-cholatrien-22-säure

Zu einem Gemisch aus 2,705 g (8 mMol) 3-Oxo-23,24-dinor-1,4,9(11),17(20)-cholatetraen-22-säure, 50 ml Pyridin und 3 ml 0,5 M Natriumwolframatlösung werden bei 60 °C unter Rühren 3 ml 30 %ige Wasserstoffperoxydlösung tropfenweise zugegeben. Nach 15 Minuten wird das Reaktionsgemisch abgekühlt und in 500 ml 0,25 %ige kalte Natriumsulfitlösung gegossen. Der pH-Wert wird mit 6M Salzsäure auf 3 eingestellt. Das ausgefallene Produkt wird abfiltriert, mit verdünnter Salzsäure und dann mit Wasser gewaschen und über Phosphorpentoxyd getrocknet. Man erhält 2,7 g 3-Oxo-17α,20-epoxy-23,24-dinor-1,4,9(11)-cholatrien-22-säure, die nach Umkristallisieren aus Aceton bei 193-195 °C schmilzt.

| Elementaranalyse für $C_{22}H_{26}O_4$ (M = 354,45): | | |
|---|---|---|
| berechnet, %: | C 74,55 | H 7,39 |
| gefunden, %: | C 74,59 | H 7,38 |

UV (Äthanol): $\lambda_{max}$: 240 nm;
IR (KBr, $cm^{-1}$): $\nu_{OH}$ 3325 (breit); $\nu_{C=O}$ 1750, 1665; $\nu_{C=C}$ 1620;
$^1$H-HMR (DMSO-$d_6$): 7,35 (1 H-1), 6,15 (1 H-2), 5,97 (1 H-4) (AHX m, $J_{1,2}$ = 10, $J_{2,4}$ = 1,8, $J_{1,4}$ ~ 0 Hz, 5,47 (dd, J = 5,8 und 1,8 Hz, 1 H-11), 1,49 (s, 3 H-21), 1,34 (s, 3 H-19), 0,85 (s, 3 H-18).

b) 3-Oxo-17α,20-epoxy-23,24-dinor-1,4,9(11)-cholatrien-22-säureazid

Zu einer Lösung von 355 mg (1 mMol) 3-Oxo-17α,20-epoxy-23,24-dinor-1,4,9(11)-cholatrien-22-säure in 10 ml Dichlormethan und 150 µl Triäthylamin werden bei -20 °C unter Rühren tropfenweise 145 µl Chlorameisensäureisobutylester gegeben. Man läßt die Temperatur des Gemisches innerhalb von 60 Minuten auf 15 °C ansteigen und versetzt dann die Lösung des gemischten Anhydrids mit 250 mg Natriumazid und 15 mg Tetrabutylammoniumbromid in 1,5 ml Wasser. Das Reaktionsgemisch wird bei 20 °C eine halbe Stunde lang gerührt und danach mit Wasser und Dichlormethan verdünnt. Die Dichlormethanphase wird mit Wasser, dann mit verdünnter Essigsäure und anschließend mit ionenfreiem Wasser gewaschen, über wasserfreiem Natriumsulfat getrocknet und im Vakuum vorsichtig eingedampft. Nach Austausch des Lösungsmittels gegen Aceton erhält man 281 mg (84 %) 3-Oxo-17α,20-epoxy-23,24-dinor-1,4,9(11)-cholatrien-22-säureazid. Schmelzverhalten: die Kristalle verlieren bei 62-65 °C Stickstoff und schmelzen bei 154-158 °C.

| Elementaranalyse für $C_{22}H_{25}N_3O_3$ (M = 379,46): | | | |
|---|---|---|---|
| berechnet, %: | C 69,64 | H 6,64 | N 11,07 |
| gefunden, %: | C 69,84 | H 6,76 | N 11,09. |

UV (Äthanol): $\lambda_{max}$: 238 nm;
IR (KBr, $cm^{-1}$): $\nu_{Azid}$ 2155; $\nu_{C=O}$ 1715, 1660; $\nu_{C=C}$ 1605;
$^1$H-NMR (DMSO-$d_6$): 7,17 (1 H-1), 6,28 (1 H-2),6,07 (1 H-4) (AMX m, $J_{1,2}$ = 10, $J_{2,4}$ = 1,7, $J_{1,4}$ ~ 0 Hz), 5,49 (dd, J = 5,8 und 1,8 Hz, 1 H-11), 1,61 (s, 3 H-21), 1,42 (s, 3 H-19), 0,93 (s, 3 H-18).

c) 17α-Hydroxy-1,4,9(11)-pregnatrien-3,20-dion

Zu einem Gemisch aus 355 mg (1 mMol) 3-Oxo-17α,20-epoxy-23,24-dinor-1,4,9(11)-cholatrien-22-säure, 15 ml Dichlormethan und 140 µl Triäthylamin werden bei -15 °C unter Rühren 145 µl Chlorameisensäureisobutylester gegeben. Man läßt die Temperatur des Reaktionsgemisches innerhalb von 60 Minuten auf 15 °C ansteigen und versetzt das Gemisch dann mit der Lösung von 150 mg Natriumazid und 15 mg Tetrabutylammoniumbromid in 1,5 ml Wasser. Das Gemisch wird bei 20 °C eine halbe Stunde lang gerührt und dann mit Wasser und Dichlormethan verdünnt. Die Dichlormethanphase wird erst mit Wasser, dann mit verdünnter Essigsäure gewaschen und anschließend ungetrocknet im Vakuum vorsichtig bis zum Beginn der Kristallisation eingeengt. Dann wird das kochende Gemisch von 10 ml Äthanol, 5 ml Methanol und 10 ml 50 %iger Essigsäure zugesetzt, das Reaktionsgemisch wird am Rückfluß 3 Stunden lang gekocht, abgekühlt und mit 100 ml Dichlormethan verdünnt. Die organische Phase wird mit Wasser gewaschen, über wasserfreiem Natriumsulfat getrocknet und dann eingedampft. Das Rohprodukt (323 mg) wird an einer mit Silikagel gefüllten Säule chromatographisch gereinigt (Elutionsmittel: Toluol und Aceton 92:8). Das reine 17α-Hydroxy-1,4,9(11)-pregna-trien-3,20-dion schmilzt bei 228-231 °C (226-230 °C nach H. Reimann et al.:

J. Org. Chem. 26, 866 /1961/).

| Elementaranalyse für $C_{21}H_{26}O_3$ (M = 326,43): | | |
|---|---|---|
| berechnet, %:<br>gefunden, %: | C 77,27<br>C 77,46 | H 8,03<br>H 8,24 |

$[\alpha]_D$: -21° (c = 1, Chloroform)
UV (Äthanol): $\lambda_{max}$: 240 nm;
IR (KBr, $cm^{-1}$): $\nu_{OH}$ 3350 (breit), $\nu_{C=O}$ 1705, 1665; $\nu_{C=C}$ 1615;
$^1$H-NMR ($CDCl_3$, $\delta$): 7,18 (1 H-1), 6,29 (1 H-2),6,09 (1 H-4) (AMX m, $J_{1,2}$ = 10, $J_{2,4}$ = 1,8, $J_{1,4}$ ~ 0 Hz), 5,56 (dd, J = 5,8 und 1,8 Hz, 1 H-11), 2,30 (s, 3 H-21), 1,42 (s, 3 H-19), 0,74 (s, 3 H-18).

**Beispiel 7**

Herstellung von 3β,17α-Dihydroxy-5-pregnen-20-on aus 3β-Hydroxy-23,24-dinor-5,17(20)-choladien-22-säure

a) 3β-Hydroxy-17α,20-epoxy-23,24-dinor-5-cholen-22-säure

Zu einem Gemisch aus 518 mg (1,5 mMol) 3β-Hydroxy-23,24-dinor-5,17(20)-choladien-22-säure, 7 ml Pyridin und 0,4 ml 0,5 M Natriumwolframatlösung werden bei 60 °C unter Rühren tropfenweise 0,8 ml 30 %ige Wasserstoffperoxydlösung gegeben. Nach 15 Minuten wird das Reaktionsgemisch abgekühlt und in 100 ml 2 %ige kalte Salzsäure gegossen. Das ausgefallene Produkt wird abfiltriert, mit Wasser gewaschen und über Phosphorpentoxyd getrocknet. Das aus Methanol umkristallisierte Produkt schmilzt bei 173-175 °C.

| Elementaranalyse für $C_{22}H_{32}O_4$ (M = 360,50): | | |
|---|---|---|
| berechnet, %:<br>gefunden, %: | C 73,30<br>C 73,18 | H 8,95<br>H 9,01. |

IR [KBr] ($cm^{-1}$): $\nu_{OH}$ 3440, 2620 (breit); $\nu_{C=O}$ 1725;
$^1$H-NMR (DMSO-$d_6$, $\delta$): 5,26 (d, $J_{6,7}$ = 4,6 Hz, 1 H-6), 3,38 (m, 1 H-3), 1,52 (s, 3 H-21), 0,94 (s, 3 H-19), 0,88 (s, 3 H-18).

b) 3β-Hydroxy-17α,20-epoxy-23,24-dinor-5-cholen-22-säureazid

Zu einem Gemisch aus 361 mg (1 mMol) 3β-Hydroxy-17α,20-epoxy-23,24-dinor-5-cholen-22-säure, 10 ml Dichlormethan und 140 µl Triäthylamin werden bei -20 °C 135 µl Chlorameisensäureäthylester gegeben. Die Lösung wird eine Stunde lang gerührt, wobei die Temperatur allmählich auf 15 °C ansteigt. Zu der Lösung des gemischten Anhydrids wird die Lösung von 200 mg Natriumazid und 15 mg Tetrabutylammoniumbromid in 1,5 ml Wasser gegeben. Das Gemisch wird bei 20 °C noch eine halbe Stunde lang gerührt und dann mit Dichlormethan und Wasser verdünnt. Die organische Phase wird zuerst mit Wasser, dann mit verdünnter Essigsäure und danach wieder mit Wasser gewaschen, über wasserfreiem Natriumsulfat getrocknet und im Vakuum eingeengt. Das Lösungsmittel wird gegen Methanol ausgetauscht, wobei das Produkt kristallisiert. Fp.: 87-88 °C.

| Elementaranalyse für $C_{22}H_{31}N_3O_3$ (M = 385,51): | | | |
|---|---|---|---|
| berechnet, %:<br>gefunden, %: | C 68,54<br>C 68,24 | H 8,11<br>H 8,16 | N 10,90<br>N 10,66 |

IR [KBr] ($cm^{-1}$): $\nu_{OH}$ 3460 (breit); $\nu_{Azid}$ 2140;
$\nu_{C=O}$ 1705;
$^1$H-NMR ($CDCl_3$, $\delta$): 5,32 (d, $J_{6,7}$ = 4,6 Hz, 1 H-6), 3,51 (m, 1 H-3), 1,62 (s, 3 H-21), 0,99 (s, 3 H-19), 0,91 (s, 3 H-18).

c) 3$\beta$,17$\alpha$-Dihydroxy-5-pregnen-20-on

Zu einer Lösung von 77 mg (0,2 mMol) 3$\beta$-Hydroxy-17$\alpha$,20-epoxy-23,24-dinor-5-cholen-22-säureazid in 200 $\mu$l Chloroform wird die kochende Lösung von 3 ml Isopropanol und 3 ml 25 %iger Essigsäure gegossen. Das Reaktionsgemisch wird am Rückfluß 7 Stunden lang gekocht und dann im Vakuum eingedampft. Das Rohprodukt wird an Silikagel mit einem 9:1-Gemisch von Toluol und Aceton chromatographiert. Das reine 3$\beta$,17$\alpha$-Dihydroxy-5-pregnen-20-on schmilzt bei 267-271 °C (271-273 °C nach P. Hegner et al.: Helv. Chim. Acta 24, 828 /1941/).

| Elementaranalyse für $C_{21}H_{32}O_3$ (M = 332,48): | | |
|---|---|---|
| berechnet, %: | C 75,86 | H 9,70 |
| gefunden, %: | C 75,78 | H 9,75 |

IR [KBr] ($cm^{-1}$): $\nu_{OH}$ 3340 (breit); $\nu_{C=O}$ 1710;
$^1$H-NMR (DMSO-$d_6$, $\delta$): 5,27 (d, $J_{6,7}$ = 4,6 Hz, 1 H-6), 3,27 (m, 1 H-3), 2,09 (s, 3 H-21), 0,94 (s, 3 H-19), 0,50 (s, 3 H-18).

## Patentansprüche

**1.** 3-(Oxo)- oder 3-(hydroxy)-substituierte 17$\alpha$,20-(Epoxy)-pregnan-20-carbonsäure- beziehungsweise -isocyanat-Derivate der allgemeinen Formel

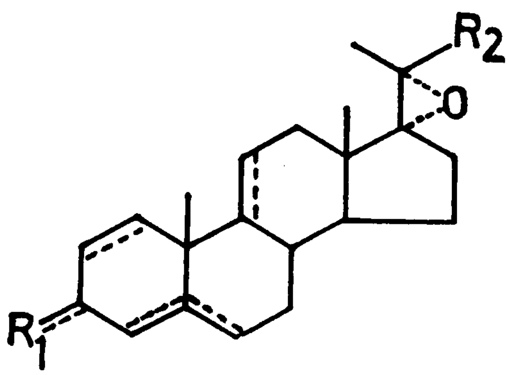

I ,

worin

| | |
|---|---|
| $R_1$ | für eine Hydroxy- oder Oxogruppe steht, |
| $R_2$ | eine Carboxylgruppe der Formel |

$$-\overset{O}{\overset{\|}{C}}-OH,$$

eine Carbonsäureanhydridgruppe, insbesondere der Formel

$$-\overset{O}{\overset{\|}{C}}-O-\overset{O}{\overset{\|}{C}}-O-C_{1-4}\text{-Alkyl},$$

eine Carbonsäureazidogruppe der Formel

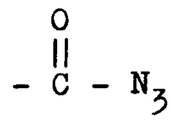

oder eine Isocyanatgruppe der Formel - N = C = O bedeutet und

die gestrichelten Linien gegebenenfalls vorhandene weitere chemische Bindungen darstellen, wobei nicht gleichzeitig in den 4(5)- und 5(6)- Stellungen Doppelbindungen vorliegen können.

**2.** 3-(Oxo)-17α,20-(epoxy)-23,24-di-(nor)-4-cholen-22-säure.

**3.** 3-(Oxo)-17α,20-(epoxy)-23,24-di-(nor)-4,9(11)-choladien-22-säure.

**4.** 3-(Oxo)-17α,20-(epoxy)-23,24-di-(nor)-1,4,9(11)-cholatrien-22-säure.

**5.** 3β-(Hydroxy)-17α,20-(epoxy)-23,24-di-(nor)-5-cholen-22-säure.

**6.** 3-(Oxo)-17α,20-(epoxy)-23,24-di-(nor)-4-cholen-22-säureazid.

**7.** 3-(Oxo)-17α,20-(epoxy)-23,24-di-(nor)-4,9(11)-choladien-22-säureazid.

**8.** 3-(Oxo)-17α,20-(epoxy)-23,24-di-(nor)-1,4,9(11)-cholatrien-22-säureazid.

**9.** 3β-(Hydroxy)-17α,20-(epoxy)-23,24-di-(nor)-5-cholen-22-säureazid.

**10.** Verwendung der 3-(oxo)- beziehungsweise 3-(hydroxy)-substituierten 17α,20-(Epoxy)-pregnan-20-carbonsäure-beziehungsweise -isocyanat-Derivate der allgemeinen Formel I, bei welchen $R_2$ für eine Isocyanatgruppe oder Carbonsäureazidogruppe steht, zur Herstellung von 3-(oxo)- oder 3-(hydroxy)-substituierten 17α-(Hydroxy)-20-(oxo)-pregnan-Derivaten der allgemeinen Formel

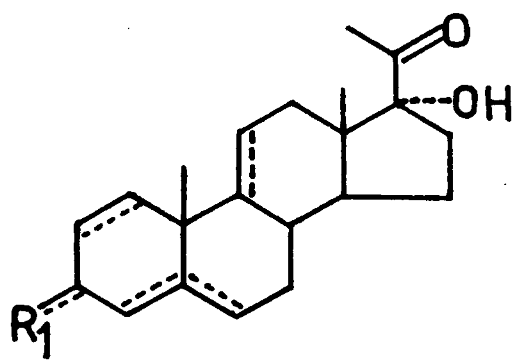

II ,

worin

$R_1$ und die gestrichelten Linien die im Anspruch 1 angegebenen Bedeutungen haben,

durch Umsetzen mit Mineralsäuren oder organischen Säuren in wasserhaltigen organischen Lösungsmitteln.

**11.** Verfahren zur Herstellung der 3-(oxo)- oder 3-(hydroxy)-substituierten 17α-(Hydroxy)-20-(oxo)-pregnan-Derivaten der allgemeinen Formel

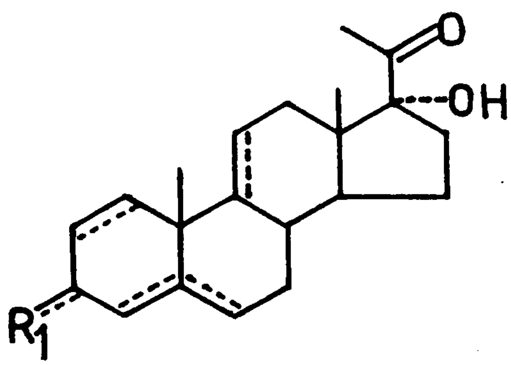

II ,

worin

$R_1$ und die gestrichelten Linien die im Anspruch 1 angegebenen Bedeutungen haben,

dadurch gekennzeichnet, daß man

a) in situ hergestellte 3-(oxo)- oder 3-(hydroxy)-substituierte 17α,20-(Epoxy)-pregnan-20-isocyanat-Derivate der allgemeinen Formel I, bei welchen $R_2$ für eine Isocyanatgruppe steht, in wasserhaltigen organischen Lösungsmitteln mit Mineralsäuren oder organischen Säuren oder solche Säuren liefernden Reagenzien umsetzt oder

b) 3-(oxo)- oder 3-(hydroxy)-substituierte 17α,20-(Epoxy)-pregnan-20-carbonsäureazid-Derivate der allgemeinen Formel I, bei welchen $R_2$ für eine Carbonsäureazidogruppe steht, in wasserhaltigen organischen Lösungsmitteln bei Temperaturen von mindestens 40°C mit Mineralsäuren oder organischen Säuren oder solche Säuren liefernden Reagenzien umsetzt oder

c) aus 3-(oxo)- oder 3-(hydroxy)-substituierten 17α,20-(Epoxy)-pregnan-20-carbonsäure-Derivaten der allgemeinen Formel I, bei welchen $R_2$ für eine Carboxylgruppe steht, hinsichtlich der Carboxylfunktion reaktionsfähige Derivate herstellt, diese mit Azidionen enthaltenden Salzen zu 3-(oxo) oder 3-(hydroxy)-substituierten 17α,20-(Epoxy)-pregnan-20-carbonsäureazid-Derivaten der allgemeinen Formel I, bei welchen $R_2$ für eine Carbonsäureazidogruppe steht, umsetzt und die letzteren in wasserhaltigen organischen Lösungsmitteln bei Temperaturen von mindestens 40 °C mit Mineralsäuren oder organischen Säuren umsetzt

oder

d) 3-(oxo)- oder 3-(hydroxy)-substituierte 23,24-Di-(nor)-17(20)-cholensäure-Derivate der allgemeinen Formel

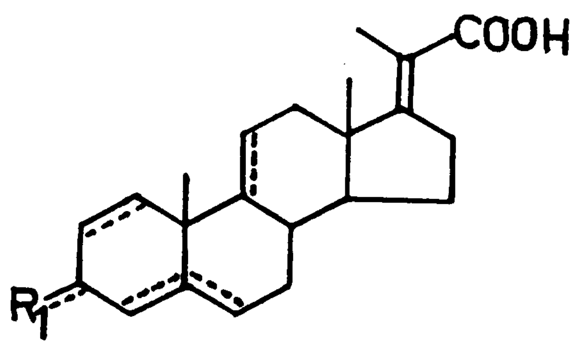

III ,

worin

$R_1$ und die gestrichelten Linien die im Anspruch 1 angegebenen Bedeutungen haben,

in wasserhaltigen organischen Lösungsmitteln in Gegenwart von Salzen von Sauerstoffsäuren oder Persäuren oder Polysäuren von Übergangsmetallen oder mit tertiären Aminen oder Säureamiden gebildeten Komplexen von solchen Übergangsmetall-Persäure- oder Polysäuresalzen mit Wasserstoffperoxyd umsetzt, aus den erhaltenen 3-(oxo)- oder 3-(hydroxy)-substituierten 17α,20-(Epoxy)-pregnan-20-carbonsäure-Derivaten der allgemeinen Formel I, bei welchen $R_2$ für eine Carboxylgruppe steht, reaktionsfähige Derivate herstellt, diese mit Azidionen enthaltenden Salzen zu 3-(oxo)- oder 3-(hydroxy)-substituierten 17α,20-(Epoxy)-pregnan-20-carbonsäureazid-Derivaten der allgemeinen Formel I, bei welchen $R_2$ für eine Carbonsäureazidogruppe steht, umsetzt und die letzteren in

wasserhaltigen organischen Lösungsmitteln bei Temperaturen von mindestens 40 °C mit Mineralsäuren oder organischen Säuren umsetzt.

**12.** Verfahren nach Anspruch 11 a), dadurch gekennzeichnet, daß man die Umsetzung der 3-(oxo)- oder 3-(hydroxy)-substituierten 17α,20-(Epoxy)-pregnan-20-isocyanat-Derivate der allgemeinen Formel I in aliphatischen Alkoholen mit 1 bis 5 Kohlenstoffatom(en) oder N,N-Di-(methyl)-formamid mit wäßriger Essigsäure als organischer Säure durchführt.

**13.** Verfahren nach Anspruch 11 b), dadurch gekennzeichnet, daß man die Umsetzung der 3-(oxo)- oder 3-(hydroxy)-substituierten 17α,20-(Epoxy)-pregnan-20-carbonsäureazid-Derivate der allgemeinen Formel I in aliphatischen Alkoholen mit 1 bis 5 Kohlenstoffatom(en) oder in Dimethylformamid mit wäßriger Essigsäure als organischer Säure bei Temperaturen von 40°C bis zur Siedetemperatur des verwendeten Lösungsmittelmediums durchführt.

**14.** Verfahren nach Anspruch 11 c) oder d), dadurch gekennzeichnet, daß man aus den 3-(oxo)- oder 3-(hydroxy)-substituierten 17α,20-(Epoxy)-pregnan-20-carbonsäure-Derivaten der allgemeinen Formel I als reaktionsfähige Derivate gemischte Anhydride herstellt, diese, gegebenenfalls ohne zwischenzeitliches Isolieren, mit Alkalimetallaziden als Azidionen enthaltenden Salzen umsetzt und die erhaltenen 3-(oxo)- oder 3-(hydroxy)-substituierten 17α,20-(Epoxy)-pregnan-20-carbonsäureazid-Derivate der allgemeinen Formel I, gegebenenfalls ohne zwischenzeitliches Isolieren, zu den entsprechenden 3-(oxo)- oder 3-(hydroxy)-substituierten 17α-(Hydroxy)-20-(oxo)-pregnan-Derivaten der allgemeinen Formel II umsetzt.

**15.** Verfahren nach Anspruch 11 c) oder d) oder 14, dadurch gekennzeichnet, daß man als gemischte Anhydride solche der allgemeinen Formel I, bei welchen $R_2$ für eine Carbonsäureanhydridgruppe der Formel

$$-\overset{\overset{\displaystyle O}{\|}}{C}-O-\overset{\overset{\displaystyle O}{\|}}{C}-O-C_{1-4}\text{-Alkyl}$$

steht, mit $C_1$-$C_4$-Alkylestern der Chlorameisensäure als Reagens herstellt.

**16.** Verfahren nach Anspruch 11 d), dadurch gekennzeichnet, daß man bei der Umsetzung der 3-(oxo)- oder 3-(hydroxy)-substituierten 23,24-Di-(nor)-17(20)-cholensäure-Derivate der allgemeinen Formel III zu den 3-(oxo)- oder 3-(hydroxy)-substituierten 17α,20-(Epoxy)-pregnan-20-carbonsäure-Derivaten der allgemeinen Formel I als organische Lösungsmittel Stickstoff enthaltende, insbesondere Pyridin, N,N-Di-(methyl)-formamid oder Hexamethylphosphorsäuretriamid, verwendet und die Umsetzung bei Temperaturen von 0 bis 100°C durchführt.

**17.** Verfahren nach Anspruch 11 d) oder 16, dadurch gekennzeichnet, daß man als Salze von Sauerstoffsäuren oder Persäuren oder Polysäuren von Übergangsmetallen $MoO_4^{--}$- oder $WO_4^{--}$-Ionen oder deren peroxydierte Formen enthaltende Salze, insbesondere Ammoniumparamolybdat oder Natriumwolframat, verwendet.

**18.** Verfahren zur Herstellung der 3-(oxo)- oder 3-(hydroxy)-substituierten 17α,20-(Epoxy)-pregnan-20-carbonsäure- beziehungsweise -isocyanat-Derivate der allgemeinen Formel I, dadurch gekennzeichnet, daß man

a) 3-(oxo)- oder 3-(hydroxy)-substituierte 23,24-Di-(nor)-17(20)-cholensäure-Derivate der allgemeinen Formel III in wasserhaltigen organischen Lösungsmitteln in Gegenwart von Salzen von Sauerstoffsäuren oder Persäuren oder Polysäuren von Übergangsmetallen oder mit tertiären Aminen oder Säureamiden gebildeten Komplexen von solchen Übergangsmetall-Persäure- oder Polysäuresalzen mit Wasserstoffperoxyd umsetzt sowie gegebenenfalls aus den erhaltenen 3-(oxo)- oder 3-(hydroxy)-substituierten 17α,20-(Epoxy)-pregnan-20-carbonsäure-Derivaten der allgemeinen Formel I, bei welchen $R_2$ für eine Carboxylgruppe steht, reaktionsfähige Derivate herstellt und gegebenenfalls diese

mit Azidionen enthaltenden Salzen zu 3-(oxo)- oder 3-(hydroxy)-substituierten 17α,20-(Epoxy)-pregnan-20-carbonsäureazid-Derivaten der allgemeinen Formel I, bei welchen $R_2$ für eine Carbonsäureazidogruppe steht, umsetzt oder

b) 3-(oxo)- oder 3-(hydroxy)-substituierte 17α,20-(Epoxy)-pregnan-20-carbonsäurehydrazid-Derivate (allgemeine Formel I mit der Abwandlung, daß $R_2$ eine Carbonsäurehydrazidogruppe der Formel

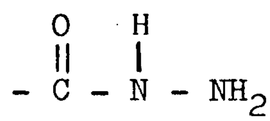

bedeutet) zu 17α,20-(Epoxy)-pregnan-20-carbonsäureazid-Derivaten der allgemeinen Formel I, bei welchen $R_2$ für eine Carbonsäureazidogruppe steht, nitrosiert oder

c) 3-(oxo)- oder 3-(hydroxy)-substituierte 17α,20-(Epoxy)-pregnan-20-carbonsäurehalogenid-Derivate, insbesondere 17α,20-(Epoxy)-pregnan-20-carbonsäurechlorid-Derivate (allgemeine Formel I mit der Abwandlung, daß

$R_2$ eine Carbonsäurehalogenidgruppe der allgemeinen Formel

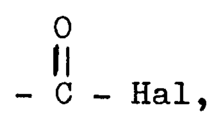

insbesondere Carbonsäurechloridgruppe der Formel

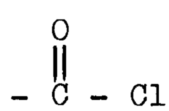

bedeutet)

mit Alkalimetallaziden zu 17α,20-(Epoxy)-pregnan-20-carbonsäureazid-Derivaten der allgemeinen Formel I, bei welchen $R_2$ für eine Carbonsäureazidogruppe steht, umsetzt oder

d) 3-(oxo)- oder 3-(hydroxy)-substituierte 17α,20-(Epoxy)-pregnan-20-carbonsäureazid-Derivate der allgemeinen Formel I, bei welchen $R_2$ für eine Carbonsäureazidogruppe steht, in der Wärme, im allgemeinen bei Temperaturen von mindestens 40°C, zu 3-(oxo)--oder 3-(hydroxy)-substituierten 17α,20-(Epoxy)-pregnan-20-isocyanat-Derivaten umlagert.

**Patentansprüche für folgende Vertragsstaaten: ES, GR**

**1.** Verfahren zur Herstellung von 3-(oxo)- oder 3-(hydroxy)-substituierten 17α,20-(Epoxy)-pregnan-20-carbonsäure-beziehungsweise -isocyanat-Derivaten der allgemeinen Formel

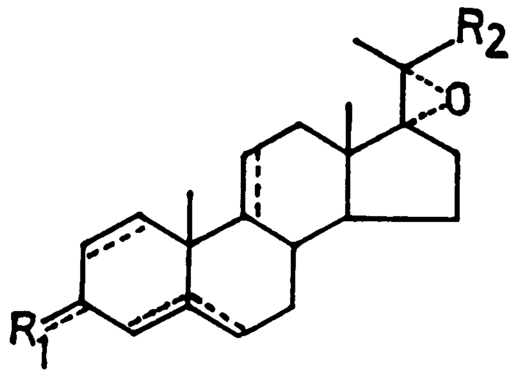

I ,

worin

| | |
|---|---|
| $R_1$ | für eine Hydroxy- oder Oxogruppe steht, |
| $R_2$ | eine Carboxylgruppe der Formel |

$$-\overset{\overset{\displaystyle O}{\|}}{C}-OH,$$

eine Carbonsäureanhydridgruppe, insbesondere der Formel

$$-\overset{\overset{\displaystyle O}{\|}}{C}-O-\overset{\overset{\displaystyle O}{\|}}{C}-O-C_{1-4}\text{-Alkyl},$$

eine Carbonsäureazidogruppe der Formel

$$-\overset{\overset{\displaystyle O}{\|}}{C}-N_3$$

oder eine Isocyanatgruppe der Formel - N = C = O bedeutet und

| | |
|---|---|
| die gestrichelten Linien | gegebenenfalls vorhandene weitere chemische Bindungen darstellen, wobei nicht gleichzeitig in den 4(5)- und 5(6)-Stellungen Doppelbindungen vorliegen können, dadurch gekennzeichnet, daß man |

a) 3-(oxo)- oder 3-(hydroxy)-substituierte 23,24-Di-(nor)-17(20)-cholensäure-Derivate der allgemeinen Formel III in wasserhaltigen organischen Lösungsmitteln in Gegenwart von Salzen von Sauerstoffsäuren oder Persäuren oder Polysäuren von Übergangsmetallen oder mit tertiären Aminen oder Säureamiden gebildeten Komplexen von solchen Übergangsmetall-Persäure- oder Polysäuresalzen mit Wasserstoffperoxyd umsetzt sowie gegebenenfalls aus den erhaltenen 3-(oxo)- oder 3-(hydroxy)-substituierten 17$\alpha$,20-(Epoxy)-pregnan-20-carbonsäure-Derivaten der allgemeinen Formel I, bei welchen $R_2$ für eine Carboxylgruppe steht, reaktionsfähige Derivate herstellt und gegebenenfalls diese mit Azidionen enthaltenden Salzen zu 3-(oxo)- oder 3-(hydroxy)-substituierten 17$\alpha$,20-(Epoxy)-pregnan-20-carbonsäureazid-Derivaten der allgemeinen Formel I, bei welchen $R_2$ für eine Carbonsäureazidogruppe steht, umsetzt oder

b) 3-(oxo)- oder 3-(hydroxy)-substituierte 17$\alpha$,20-(Epoxy)-pregnan-20-carbonsäurehydrazid-Derivate (allgemeine Formel I mit der Abwandlung, daß $R_2$ eine Carbonsäurehydrazidogruppe der Formel

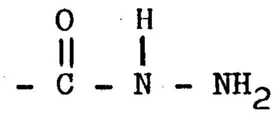

bedeutet) zu 17α,20-(Epoxy)-pregnan-20-carbonsäureazid-Derivaten der allgemeinen Formel I, bei welchen $R_2$ für eine Carbonsäureazidogruppe steht, nitrosiert oder

c) 3-(oxo)- oder 3-(hydroxy)-substituierte 17α,20-(Epoxy)-pregnan-20-carbonsäurehalogenid-Derivate, insbesondere 17α,20-(Epoxy)-pregnan-20-carbonsäurechlorid-Derivate (allgemeine Formel I mit der Abwandlung, daß

$R_2$ eine Carbonsäurehalogenidgruppe der allgemeinen Formel

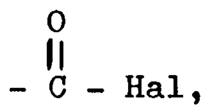

insbesondere Carbonsäurechloridgruppe der Formel

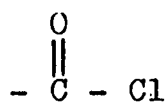

bedeutet)

mit Alkalimetallaziden zu 17α,20-(Epoxy)-pregnan-20-carbonsäureazid-Derivaten der allgemeinen Formel I, bei welchen $R_2$ für eine Carbonsäureazidogruppe steht, umsetzt oder

d) 3-(oxo)- oder 3-(hydroxy)-substituierte 17α,20-(Epoxy)-pregnan-20-carbonsäureazid-Derivate der allgemeinen Formel I, bei welchen $R_2$ für eine Carbonsäureazidogruppe steht, in der Wärme, im allgemeinen bei Temperaturen von mindestens 40 °C, zu 3-(oxo)--oder 3-(hydroxy)-substituierten 17α,20-(Epoxy)-pregnan-20-isocyanat-Derivaten umlagert.

2. Verfahren nach Anspruch 1, dadurch gekennzeichnet, daß man 3-(Oxo)-17α,20-(epoxy)-23,24-di-(nor)-4-cholen-22-säure herstellt.

3. Verfahren nach Anspruch 1, dadurch gekennzeichnet, daß man 3-(Oxo)-17α,20-(epoxy)-23,24-di-(nor)-4,9(11)-choladien-22-säure herstellt.

4. Verfahren nach Anspruch 1, dadurch gekennzeichnet, daß man 3-(Oxo)-17α,20-(epoxy)-23,24-di-(nor)-1,4,9(11)-cholatrien-22-säure herstellt.

5. Verfahren nach Anspruch 1, dadurch gekennzeichnet, daß man 3β-(Hydroxy)-17α,20-(epoxy)-23,24-di-(nor)-5-cholen-22-säure herstellt.

6. Verfahren nach Anspruch 1, dadurch gekennzeichnet, daß man 3-(Oxo)-17α,20-(epoxy)-23,24-di-(nor)-4-cholen-22-säureazid herstellt.

7. Verfahren nach Anspruch 1, dadurch gekennzeichnet, daß man 3-(Oxo)-17α,20-(epoxy)-23,24-di-(nor)-4,9(11)-choladien-22-säureazid herstellt.

8. Verfahren nach Anspruch 1, dadurch gekennzeichnet, daß man 3-(Oxo)-17α,20-(epoxy)-23,24-di-(nor)-1,4,9(11)-cholatrien-22-säureazid herstellt.

9. Verfahren nach Anspruch 1, dadurch gekennzeichnet, daß man 3β-(Hydroxy)-17α,20-(epoxy)-23,24-di-(nor)-5-cholen-22-säureazid herstellt.

10. Verwendung der 3-(oxo)- beziehungsweise 3-(hydroxy)-substituierten 17α,20-(Epoxy)-pregnan-20-carbonsäure-beziehungsweise -isocyanat-Derivate der allgemeinen Formel I, bei welchen $R_2$ für eine Isocyanatgruppe oder Carbonsäureazidogruppe steht, zur Herstellung von 3-(oxo)- oder 3-(hydroxy)-substituierten 17α-(Hydroxy)-20-(oxo)-pregnan-Derivaten der allgemeinen Formel

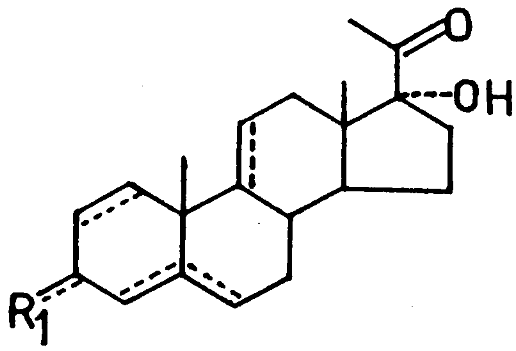

II ,

worin

$R_1$ und die gestrichelten Linien die im Anspruch 1 angegebenen Bedeutungen haben,

durch Umsetzen mit Mineralsäuren oder organischen Säuren in wasserhaltigen organischen Lösungsmitteln.

**11.** Verfahren zur Herstellung der 3-(oxo)- oder 3-(hydroxy)-substituierten 17α-(Hydroxy)-20-(oxo)-pregnan-Derivaten der allgemeinen Formel

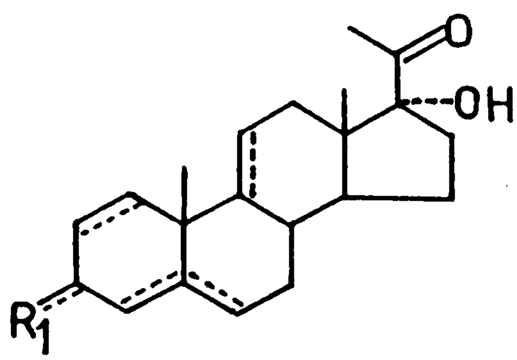

II ,

worin

$R_1$ und die gestrichelten Linien die im Anspruch 1 angegebenen Bedeutungen haben,

dadurch gekennzeichnet, daß man

a) in situ hergestellte 3-(oxo)- oder 3-(hydroxy)-substituierte 17α,20-(Epoxy)-pregnan-20-isocyanat-Derivate der allgemeinen Formel I, bei welchen $R_2$ für eine Isocyanatgruppe steht, in wasserhaltigen organischen Lösungsmitteln mit Mineralsäuren oder organischen Säuren oder solche Säuren liefernden Reagenzien umsetzt oder

b) 3-(oxo)- oder 3-(hydroxy)-substituierte 17α,20-(Epoxy)-pregnan-20-carbonsäureazid-Derivate der allgemeinen Formel I, bei welchen $R_2$ für eine Carbonsäureazidogruppe steht, in wasserhaltigen organischen Lösungsmitteln bei Temperaturen von mindestens 40°C mit Mineralsäuren oder organischen Säuren oder solche Säuren liefernden Reagenzien umsetzt oder

c) aus 3-(oxo)- oder 3-(hydroxy)-substituierten 17α,20-(Epoxy)-pregnan-20-carbonsäure-Derivaten der allgemeinen Formel I, bei welchen $R_2$ für eine Carboxylgruppe steht, hinsichtlich der Carboxylfunktion reaktionsfähige Derivate herstellt, diese mit Azidionen enthaltenden Salzen zu 3-(oxo)- oder 3-(hydroxy)-substituierten 17α,20-(Epoxy)-pregnan-20-carbonsäureazid-Derivaten der allgemeinen Formel I, bei welchen $R_2$ für eine Carbonsäureazidogruppe steht, umsetzt und die letzteren in wasserhaltigen organischen Lösungsmitteln bei Temperaturen von mindestens 40 °C mit Mineralsäuren oder organischen Säuren umsetzt

oder

d) 3-(oxo)- oder 3-(hydroxy)-substituierte 23,24-Di-(nor)-17(20)-cholensäure-Derivate der allgemeinen Formel

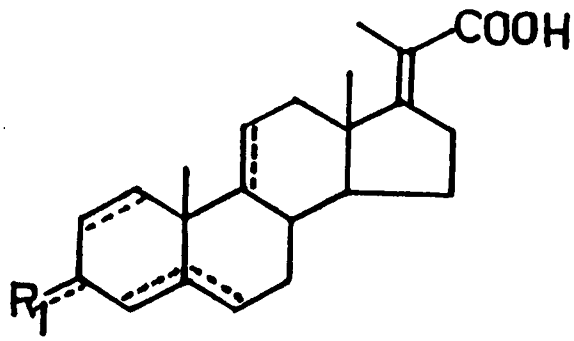

III ,

worin

$R_1$ und die gestrichelten Linien die im Anspruch 1 angegebenen Bedeutungen haben,

in wasserhaltigen organischen Lösungsmitteln in Gegenwart von Salzen von Sauerstoffsäuren oder Persäuren oder Polysäuren von Übergangsmetallen oder mit tertiären Aminen oder Säureamiden gebildeten Komplexen von solchen Übergangsmetall-Persäure- oder Polysäuresalzen mit Wasserstoffperoxyd umsetzt, aus den erhaltenen 3-(oxo)- oder 3-(hydroxy)-substituierten 17$\alpha$,20-(Epoxy)-pregnan-20-carbonsäure-Derivaten der allgemeinen Formel I, bei welchen $R_2$ für eine Carboxylgruppe steht, reaktionsfähige Derivate herstellt, diese mit Azidionen enthaltenden Salzen zu 3-(oxo)- oder 3-(hydroxy)-substituierten 17$\alpha$,20-(Epoxy)-pregnan-20-carbonsäureazid-Derivaten der allgemeinen Formel I, bei welchen $R_2$ für eine Carbonsäureazidogruppe steht, umsetzt und die letzteren in wasserhaltigen organischen Lösungsmitteln bei Temperaturen von mindestens 40 °C mit Mineralsäuren oder organischen Säuren umsetzt.

**12.** Verfahren nach Anspruch 11 a), dadurch gekennzeichnet, daß man die Umsetzung der 3-(oxo)- oder 3-(hydroxy)-substituierten 17$\alpha$,20-(Epoxy)-pregnan-20-isocyanat-Derivate der allgemeinen Formel I in aliphatischen Alkoholen mit 1 bis 5 Kohlenstoffatom(en) oder N,N-Di-(methyl)-formamid mit wäßriger Essigsäure als organischer Säure durchführt.

**13.** Verfahren nach Anspruch 11 b), dadurch gekennzeichnet, daß man die Umsetzung der 3-(oxo)- oder 3-(hydroxy)-substituierten 17$\alpha$,20-(Epoxy)-pregnan-20-carbonsäureazid-Derivate der allgemeinen Formel I in aliphatischen Alkoholen mit 1 bis 5 Kohlenstoffatom(en) oder in Dimethylformamid mit wäßriger Essigsäure als organischer Säure bei Temperaturen von 40°C bis zur Siedetemperatur des verwendeten Lösungsmittelmediums durchführt.

**14.** Verfahren nach Anspruch 11 c) oder d), dadurch gekennzeichnet, daß man aus den 3-(oxo)- oder 3-(hydroxy)-substituierten 17$\alpha$,20-(Epoxy)-pregnan-20-carbonsäure-Derivaten der allgemeinen Formel I als reaktionsfähige Derivate gemischte Anhydride herstellt, diese, gegebenenfalls ohne zwischenzeitliches Isolieren, mit Alkalimetallaziden als Azidionen enthaltenden Salzen umsetzt und die erhaltenen 3-(oxo)- oder 3-(hydroxy)-substituierten 17$\alpha$,20-(Epoxy)-pregnan-20-carbonsäureazid-Derivate der allgemeinen Formel I, gegebenenfalls ohne zwischenzeitliches Isolieren, zu den entsprechenden 3-(oxo)- oder 3-(hydroxy)-substituierten 17$\alpha$-(Hydroxy)-20-(oxo)-pregnan-Derivaten der allgemeinen Formel II umsetzt.

**15.** Verfahren nach Anspruch 11 c) oder d) oder 14, dadurch gekennzeichnet, daß man als gemischte Anhydride solche der allgemeinen Formel I, bei welchen $R_2$ für eine Carbonsäureanhydridgruppe der Formel

$$-\overset{\overset{\displaystyle O}{\|}}{C}-O-\overset{\overset{\displaystyle O}{\|}}{C}-O-C_{1-4}\text{-Alkyl}$$

steht, mit $C_1$-$C_4$-Alkylestern der Chlorameisensäure als Reagens herstellt.

**16.** Verfahren nach Anspruch 11 d), dadurch gekennzeichnet, daß man bei der Umsetzung der 3-(oxo)- oder 3-(hydroxy)-substituierten 23,24-Di-(nor)-17(20)-cholensäure-Derivate der allgemeinen Formel III zu den 3-(oxo)- oder 3-(hydroxy)-substituierten 17α,20-(Epoxy)-pregnan-20-carbonsäure-Derivaten der allgemeinen Formel I als organische Lösungsmittel Stickstoff enthaltende, insbesondere Pyridin, N,N-Di-(methyl)-formamid oder Hexamethylphosphorsäuretriamid, verwendet und die Umsetzung bei Temperaturen von 0 bis 100°C durchführt.

**17.** Verfahren nach Anspruch 11 d) oder 16, dadurch gekennzeichnet, daß man als Salze von Sauerstoffsäuren oder Persäuren oder Polysäuren von Übergangsmetallen $MoO_4^{--}$- oder $WO_4^{--}$-Ionen oder deren peroxydierte Formen enthaltende Salze, insbesondere Ammoniumparamolybdat oder Natriumwolframat, verwendet.